(19) Europäisches Patentamt
European Patent Office
Office européen des brevets

(11) **EP 2 366 102 B1**

(12) **EUROPÄISCHE PATENTSCHRIFT**

(45) Veröffentlichungstag und Bekanntmachung des Hinweises auf die Patenterteilung:
**06.11.2019 Patentblatt 2019/45**

(21) Anmeldenummer: **09747855.6**

(22) Anmeldetag: **06.11.2009**

(51) Int Cl.:
*B01L 3/00* *(2006.01)*      *G01N 21/84* *(2006.01)*
*G01N 21/64* *(2006.01)*      *G01N 33/52* *(2006.01)*
*G01N 33/552* *(2006.01)*

(86) Internationale Anmeldenummer:
**PCT/EP2009/064758**

(87) Internationale Veröffentlichungsnummer:
**WO 2010/052307 (14.05.2010 Gazette 2010/19)**

(54) **VORRICHTUNG UND VERFAHREN FÜR DEN NACHWEIS EINES ANALYTEN IN EINER PROBE**

DEVICE AND METHOD FOR DETECTING AN ANALYTE IN A SAMPLE

DISPOSITIF ET PROCÉDÉ DE DÉTECTION D'UN ANALYTE DANS UN ÉCHANTILLON

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **07.11.2008 EP 08168666**

(43) Veröffentlichungstag der Anmeldung:
**21.09.2011 Patentblatt 2011/38**

(73) Patentinhaber:
• **Roche Diabetes Care GmbH**
  **68305 Mannheim (DE)**
  Benannte Vertragsstaaten:
  **DE**
• **F. Hoffmann-La Roche AG**
  **4070 Basel (CH)**
  Benannte Vertragsstaaten:
  **AT BE BG CH CY CZ DK EE ES FI FR GB GR HR HU IE IS IT LI LT LU LV MC MK MT NL NO PL PT RO SE SI SK SM TR**

(72) Erfinder:
• **PETRICH, Wolfgang**
  **76669 Bad Schönborn (DE)**
• **BEDON-Gomez, Luis David**
  **Bogota (CO)**

(74) Vertreter: **Altmann Stößel Dick Patentanwälte PartG mbB**
  **Dudenstrasse 46**
  **68167 Mannheim (DE)**

(56) Entgegenhaltungen:
EP-A1- 1 733 792      EP-A2- 0 348 006
EP-A2- 0 821 235      DE-A1- 19 844 500
US-A- 4 791 461      US-A1- 2002 119 486
US-A1- 2004 071 331

**Beschreibung**

Gebiet der Erfindung

Die Erfindung betrifft eine Vorrichtung und ein Verfahren zum Nachweis mindestens eines Analyten in einer Probe. Derartige Vorrichtungen und Verfahren werden insbesondere zum Nachweis von Metaboliten in Körperflüssigkeiten eingesetzt. Ein Schwerpunkt der folgenden Erfindung liegt auf dem Nachweis von Glucose in einer Körperflüssigkeit, insbesondere Blut und/oder interstitieller Flüssigkeit. Grundsätzlich sind jedoch auch andere Arten von Proben einsetzbar, beispielsweise andere Arten von Körperflüssigkeiten, wie beispielsweise Urin oder Speichel, sowie andere Arten von Analyten, wie beispielsweise Cholesterin, Lactat, Coagulate oder Ähnliches. Neben einem Einsatzgebiet in der medizinischen Diagnostik ist grundsätzlich der Einsatz der erfindungsgemäßen Vorrichtungen und Verfahren in anderen Bereichen möglich, beispielsweise in anderen Bereichen der Naturwissenschaften und Technik, insbesondere beispielsweise in der chemischen Analytik.

Stand der Technik

In vielen Bereichen der Naturwissenschaften und Technik müssen zuverlässig und schnell ein oder mehrere Analyte qualitativ und/oder quantitativ in einer Probe, beispielsweise einer flüssigen und/oder gasförmigen Probe, nachgewiesen werden. Ein Schwerpunkt der vorliegenden Erfindung liegt auf Analysen in der medizinischen Diagnostik, insbesondere in der Diabetes-Vorsorge und der Diabetes-Behandlung. Im Rahmen einer Diabetes-Vorsorge und/oder einer Diabetes-Behandlung ist es in der Regel erforderlich, den Blutzuckergehalt mindestens einmal am Tage, in der Regel mehrfach, schnell und zuverlässig zu bestimmen, um bei Abweichungen vom Normwert entsprechende Gegenmaßnahmen ergreifen zu können. Um den Tagesablauf des Patienten durch diese Messungen nicht mehr als notwendig zu beeinträchtigen, wurden Vorrichtungen und Verfahren entwickelt, welche die Blutzuckerbestimmung nicht nur in einem klinischen Umfeld, sondern beispielsweise auch am Arbeitsplatz, zu Hause oder bei Freizeitbeschäftigungen ermöglichen. Daneben sind auch Vorrichtungen und Verfahren bekannt, welche im klinischen Umfeld eingesetzt werden.

Derartige Vorrichtungen und Verfahren beruhen in der Regel auf der Verwendung eines oder mehrerer Testelemente. Diese Testelemente sind in unterschiedlichen Formen bekannt und erhältlich, auf welche die vorliegende Erfindung insgesamt anwendbar ist. So sind beispielsweise Testelemente in Form von Teststreifen, Testbändern, Testscheiben, faltbaren Testelementen (beispielsweise nach einem so genannten Leporello-Prinzip) oder in anderen Formen bekannt. Im Folgenden wird die Erfindung im Wesentlichen unter Bezugnahme auf Teststreifen beschrieben, wobei jedoch auch andere Ausgestaltungen grundsätzlich möglich sind.

Testelemente umfassen in der Regel ein oder mehrere Testfelder mit mindestens einem Analyt-spezifischen Nachweisreagenz. Dieses Nachweisreagenz ist ausgewählt und ausgestaltet, um bei Anwesenheit des nachzuweisenden Analyten eine nachweisbare Reaktion durchzuführen. Unter einer nachweisbaren Reaktion wird dabei im Folgenden eine Reaktion verstanden, welche mittels mindestens eines physikalischen und/oder chemischen Nachweisverfahrens detektierbar ist, vorzugsweise mittels eines physikalischen Nachweisverfahrens. Beispielsweise sind Reaktionen bekannt, die auf optischem und/oder elektrochemischem Wege nachgewiesen werden können. Beispielsweise lassen sich dabei Reaktionen einsetzen, bei denen mindestens ein Nachweisstoff gebildet wird, welcher optisch und/oder elektrochemisch nachgewiesen werden kann.

So beschreibt beispielsweise EP 0 821 233 A2 einen diagnostischen Testträger mit einer Tragschicht. Auf der Tragschicht ist eine Nachweisschicht angeordnet, welche die zur Bestimmung des Analyten in einer flüssigen Probe erforderlichen Reagenzien enthält. Weiterhin umfasst der diagnostische Testträger ein die Nachweisschicht überdeckendes Netzwerk (Spreitnetz), welches größer ist als die Nachweisschicht und welches auf der Tragschicht befestigt ist. Das Netzwerk ist hydrophil, aber alleine nicht kapillaraktiv und umfasst auf den über die Nachweisschicht hinausragenden Bereichen eine inerte Abdeckung aus Proben-undurchlässigem Material.

Der in EP 0 821 233 A2 beschriebene Testträger mit dem Spreitnetz erfordert jedoch bei seiner Herstellung einen zusätzlichen Verfahrensschritt, nämlich das Aufbringen des Spreitnetzes. Zudem kann sich die Verwendung des Spreitnetzes negativ auf den Bauraum des Testträgers auswirken, denn das Spreitnetz stellt ein Flüssigkeitsreservoir für die Probe bereit, welches eine gewisse Mindestdicke nicht unterschreiten darf. In der Regel erhöht sich somit die Dicke der Testträger durch die Verwendung eines Spreitnetzes um beispielsweise 5 $\mu$m, was sich beispielsweise bei magazinierten Testelementen bereits negativ bemerkbar machen kann. Eine weitere Problematik bei der Verwendung von Spreitnetzen besteht darin, dass diese bei optischen Nachweisen die Homogenität des Nachweises beeinflussen können. So kann es beispielsweise bei Farbänderungen zu einer Abbildung der Spreitnetze kommen, also zu einer ungleichmäßigen Verfärbung. Die Verwendung von Spreitnetzen impliziert also in der Regel technische und wirtschaftliche Herausforderungen, welche unerwünscht sein können.

Weiterhin steigen bei diagnostischen Systemen, wie beispielsweise Glucose-Messsystemen, die technischen Anforderungen an bestimmte Randbedingungen der Systeme und der damit durchzuführenden Messverfahren stetig.

So besteht ein zunehmender Trend hin zu einer Miniaturisierung der Messsysteme, insbesondere hinsichtlich eines stetig sinkenden Probenvolumens. Weiterhin besteht ein wachsendes Verlangen nach Schnelligkeit in dem Messprozess, bei einer gleichzeitigen Forderung nach Robustheit. Zudem stehen die Verfahren und Vorrichtungen unter stetig wachsendem Kostendruck.

Eine Problematik, welche insbesondere durch die beschriebenen steigenden Anforderungen bedingt ist, liegt insbesondere in einer gleichmäßigen, flächigen und schnellen Verteilung (Spreiten) kleiner Blutproben auf den Testfeldern mit der Reagenzschicht auf den Testelementen, beispielsweise Glucoseteststreifen. Insbesondere bei den derzeitigen photometrischen Trockentesten äußert sich eine geringe Blutmenge in einer nur teilweisen Benetzung der Testfelder, was insbesondere durch eine in vielen Fällen nicht ausreichende Benetzbarkeit der Testfelder beziehungsweise der darin enthaltenen Reaktionsschichten verursacht wird. Netzwerke, auch als "Spreitnetze" bezeichnet, wie sie beispielsweise in EP 0 821 233 A2 beschrieben werden und welche einer Verbesserung der Benetzbarkeit dienen, ermöglichen diesbezüglich zwar eine Minderung der Problematik, garantieren selbst jedoch noch keine gleichmäßige, flächige Benetzung der Testfelder. Zudem bleibt das System, insbesondere das Testelement mit der Probe, ein offenes System, das beispielsweise von Schwankungen im Volumen der Probe, beispielsweise dem Tropfenvolumen, abhängig ist. Insbesondere, wenn diese kleinen Volumina, beispielsweise auf Spreitnetzen, flächig aufgebracht werden und somit eine große Oberfläche für die Verdunstung bieten, stellt dieser Effekt eine Herausforderung dar. Hinzu kommt in vielen Fällen eine Wölbung der Spreitnetze, welche zu Inhomogenitäten in dem Spreitverhalten der Probe führen kann. Insbesondere bei kleinen Probenvolumina äußert sich diese Inhomogenität gegebenenfalls als Unsicherheit in der Bestimmung der Glucosekonzentration. Auch ein Ablösen des Spreitnetzes von der Reagenzschicht kann diesbezüglich zu Problemen führen.

Aus dem Stand der Technik sind weiterhin mehrere Teststreifensysteme bekannt, welche als Kapillarteststreifen mit einem Folienaufbau ausgestaltet sind. So beschreibt beispielsweise DE 197 53 847 A1 ein analytisches Testelement mit einem Kapillarkanal. Das Testelement umfasst einen inerten Träger, ein Nachweiselement und einen zum kapillaren Flüssigkeitstransport befähigten Kanal mit einer Probenaufgabeöffnung und einer Entlüftungsöffnung. Der zum kapillaren Flüssigkeitstransport befähigte Kanal wird dabei zumindest teilweise vom Träger und dem Nachweiselement gebildet.

US 2003/0068 666 A1 beschreibt einen diagnostischen Trockenreagenz-Test, welcher in der Lage ist, mit einem einzigen Blutstropfen zu reagieren und sowohl Glucose als auch Betahydroxybutyrat nachzuweisen. Dabei wird unter anderem ein Sandwich-Aufbau vorgeschlagen, bei welchem durch Verwendung zweier PVC-Schichten und eines Abstandhalters eine Öffnung zur Aufnahme einer Blutprobe ausgebildet ist, innerhalb derer Reagenzienfelder ausgebildet sind.

Die aus dem Stand der Technik bekannten Testelemente, wie beispielsweise die Testelemente in DE 197 53 847 A1 oder in US 2003/0068 666 A1, weisen jedoch noch technische Herausforderungen auf. So wird beispielsweise in US 2003/0068 666 A1 versucht, über eine Optimierung der Porosität der Reagenzschicht ein Spreitverhalten zu beeinflussen. Nach wie vor bildet das dort beschriebene Testelement jedoch ein offenes System mit den oben dargestellten Problemen, also ein System, bei welchem zum einen Probenflüssigkeit verdunsten kann und bei welchem zum anderen die Schichtdicke, also bei gegebener Fläche das Volumen, nicht konstant ist. Auch bei dem in DE 197 53 847 A1 dargestellten Testelement sind Schwierigkeiten einer gleichmäßigen Benetzung des Testfelds zu beobachten. Zudem hat sich bei Messungen gezeigt, dass die Messergebnisse von der genauen zeitlichen Differenz zwischen der Probenaufgabe und dem Erreichen des Gleichgewichtszustands abhängen können.

EP 0 821 235 A2 offenbart einen ein diagnostischen Testträger enthaltend eine Tragschicht mit einer darauf angeordneten zur Bestimmung eines Analyten in flüssiger Probe erforderliche Reagenzien enthaltenden Nachweisschicht und eine die Nachweisschicht überdeckende inerte Schicht, wobei die inerte Schicht in einem solchen Abstand von der Nachweisschicht angeordnet ist, dass zwischen Nachweis- und inerter Schicht ein kapillaraktiver Flüssigkeitstransport möglich ist, dadurch gekennzeichnet, dass die inerte Schicht über die Nachweisschicht hinausreicht und in dem die Nachweisschicht überragenden Bereich so auf der Tragschicht befestigt ist, dass ein durchgehender kapillaraktiver Flüssigkeitstransport zwischen inerter und Nachweisschicht und zwischen inerter und Tragschicht möglich ist und außerdem die inerte Schicht im Bereich der Nachweisschicht mehrere Löcher enthält, durch die die zu untersuchende Probe und darin enthaltene Inhaltsstoffe auf die Nachweisschicht aufgegeben werden kann sowie dessen Verwendung zur Bestimmung eines Analyten in einer Flüssigkeit.

EP 1 733 792 A1 offenbart Vorrichtungen, welche zumindest zwei gegenüber liegende Flächen in einem Kapillarabstand umfassen, wobei wenigstens eine der Flächen dazu eingerichtet ist, mindestens einen Zielliganden in einer Menge, welche sich auf die Menge des Zielliganden in einer Probe bezieht, aus der Probe in einen Bereich zur kontrollierten Bewegung von Flüssigkeiten in, durch oder aus dem Bereich zu immobilisieren. Diese Komponenten können in ein membranhaltiges oder membranloses Assay eingebracht werden.

EP 0 348 006 A2 offenbart eine Vorrichtung zur Bereitstellung eines kontrollierten Flusses an Flüssigkeit in einer Flusszone zwischen zwei Flächen, wobei eine der Flächen eine Vielzahl von Vorsprüngen oder Erhebungen, die im Wesentlichen über die Fläche der Flusszone angeordnet sind, und eine Öffnung aufweist, welche eine Einbringung der Flüssigkeit zwischen den beiden Flächen ermöglicht. Die zweite der Flächen kann hierbei als Schicht eines Assay-Elements ausgestaltet sein.

US 2002/119486 A1 offenbart ein Element und ein Verfahren zur Bereitstellung eines Assays. Das Element verwendet Kapillarkräfte, um ein festgelegtes Volumen einer flüssigen Probe in eine Reaktionskammer, die mit einem Reagenz geladen ist, zu ziehen, wobei die Reaktion zwischen der Probe und dem Reagenz überwacht wird.

DE 198 44 500 A1 offenbart ein Verfahren und eine Vorrichtung zur photometrischen Auswertung von Testelementen mit einer Nachweiszone, das stabil gegen Positionierungstoleranzen der Nachweiszone ist, mit den Schritten Aktivierung einer ersten Lichtquelle zur Bestrahlung eines ersten Bereiches der Nachweiszone und Detektion von der Nachweiszone reflektierten oder durch die Nachweiszone transmittierten Lichts zur Generierung eines ersten Detektionssignals; Aktivierung einer zweiten Lichtquelle zur Bestrahlung eines zweiten Bereiches der Nachweiszone, der gegenüber dem ersten Bereich in Richtung der Positionierungstoleranz versetzt ist und Detektion von der Nachweiszone reflektierten oder durch die Nachweiszone transmittierten Lichtes zur Generierung eines zweiten Detektionssignals; Vergleich des ersten und des zweiten Detektionssignals und Ermittlung, ob das erste und/oder das zweite Detektionssignal durch Beleuchtung eines vollständig auf der Nachweiszone liegenden Bereiches gewonnen wurde.

US 4 791 461 A offenbart ein tragbares Analysegerät, umfassen ein Gehäuse, ein optisches System mit Lichtquelle, Detektor und einer Einrichtung zur Aktivierung des optischen Systems zur Erzeugung einer Signalantwort auf ein Testelement, Einrichtungen zur manuellen Bewegung des Testelements von einer ersten Position zu einer zweiten Position, die einem Initiationspunkt des Betriebs des optischen Systems entspricht, Einrichtungen zur Beeinflussung der Bewegung zur ersten Position zur Kontrolle der Bewegung des Testelements von der zweiten Position zur ersten Position, eine Einrichtung zur Verarbeitung der Signale und eine Einrichtung zur Darstellung der Signale.

US 2004/071331 A1 offenbart eine Vorrichtung, welche die Reflektivität von Licht dazu verwendet, um die Intensität eines getrockneten Flecks auf einer Membran, die von einem Hintergrundbereich umgeben ist, zu untersuchen, um Information über die den Fleck verursachende Probe zu gewinnen. Das von dem Fleck reflektierte Licht wird bevorzugt von zwei Fotodetektoren nachgewiesen, die ein Vielfaches von 90° gegeneinander versetzt angeordnet sind.

Aufgabe der Erfindung

Es ist daher eine Aufgabe der vorliegenden Erfindung, eine Vorrichtung und ein Verfahren zum Nachweis mindestens eines Analyten in einer Probe bereitzustellen, welche die Nachteile bekannter Vorrichtungen und Verfahren zumindest weitgehend vermeiden. Insbesondere soll eine möglichst schnelle und gleichmäßige Veränderung der optischen Eigenschaften der Probe in einem Testfeld des Testelements gewährleistet werden, und die Reproduzierbarkeit der Messergebnisse und die Unabhängigkeit der Messergebnisse vom Messzeitpunkt sollen erhöht werden.

Beschreibung der Erfindung

Diese Aufgabe wird durch eine Vorrichtung, sowie ein Verfahren mit den Merkmalen der unabhängigen Ansprüche gelöst. Vorteilhafte Weiterbildungen der Erfindung, welche einzeln oder in Kombination realisiert werden können, sind in den abhängigen Ansprüchen dargestellt. Dabei umfasst die Vorrichtung mindestens ein Testelement gemäß einer oder mehreren der im Folgenden beschriebenen Ausführungsformen und das Verfahren kann unter Verwendung einer erfindungsgemäßen Vorrichtung und eines Testelements in einer oder mehreren der beschriebenen Ausführungsformen durchgeführt werden. Insofern kann für mögliche Einzelheiten der Vorrichtung auf die Beschreibung der möglichen Details des Testelements verwiesen werden. Analog kann für das Verfahren und dessen optionale Ausgestaltungen auf die Beschreibung des Testelements und/oder die Beschreibung der Vorrichtung verwiesen werden.

Das Testelement zum Nachweis mindestens eines Analyten in einer Probe kann insbesondere zum Nachweis mindestens eines Metaboliten in einer Körperflüssigkeit eingerichtet sein. Ein besonderer Schwerpunkt der nachfolgenden Beschreibung wird dabei auf dem Nachweis von Glucose in einer Probe einer Körperflüssigkeit liegen, insbesondere in einer Blutprobe. Für Verallgemeinerungen möglicher Analyten und/oder möglicher Proben wird auf die obige Beschreibung verwiesen. Das Testelement kann dabei auf verschiedenste Weise ausgestaltet sein. Für die möglichen äußeren Erscheinungsformen des Testelements kann daher ebenfalls auf die obige Beschreibung verwiesen werden, so dass beispielsweise Teststreifen, Testbänder, Testscheiben oder andere der oben beschriebenen Erscheinungsformen eingesetzt werden können. Im Folgenden werden, ohne Beschränkung weiterer, alternativ oder zusätzlich einsetzbarer Ausführungsformen, insbesondere flache, streifenförmige Testelemente, also Teststreifen, beschrieben.

Das Testelement umfasst mindestens ein Testfeld mit einer Testfeldoberfläche. Unter einem Testfeld ist dabei ein zwei- oder dreidimensionaler Bereich des Testelements zu verstehen, welcher grundsätzlich für den, qualitativ und/oder quantitativ durchführbaren, Nachweis des Analyten einsetzbar ist. Insbesondere kann das Testfeld als Trockentestfeld ausgestaltet sein. Das Testfeld umfasst mindestens ein Nachweisreagenz, welches eingerichtet ist, um bei Anwesenheit des Analyten eine nachweisbare Reaktion durchzuführen. Diesbezüglich kann beispielsweise ebenfalls auf die obige Beschreibung verwiesen werden, beispielsweise die im eingangs genannten Stand der Technik erwähnten Nachweisreagenzien. Zusätzlich zu dem mindestens einen Nachweisereagenz, welches mindestens eine Analyt-spezifische Reaktion durchführen kann, kann das Testfeld weitere Substanzen enthalten, beispielsweise Trägerstoffe, Hilfsstoffe, Pigmente, Füllstoffe, Pufferstoffe oder Ähnliches. Zwischen weiteren Stoffen, die ebenfalls an der Reaktion zum Nachweis des Analyten beteiligt sind, und dem eigentlichen Nachweisreagenz, wird im Folgenden dabei nicht unterschieden. Insbesondere kann das Nachweisreagenz ein enzymatisches Nachweisreagenz umfassen. Als Beispiele derartiger Glucose-spezifischer enzymatischer Nachweisreagenzien sind Deoxireductase-Enzyme (z.B. GlucDOR/PQQ), Dehydrogenase-Enzyme, Oxidase-Enzyme oder ähnliche Enzyme zu nennen, beispielsweise GlucoseOxidase (GOD) oder Glucosedehydrogenase. Bei der mindestens einen nachweisbaren Reaktion handelt es sich um eine optisch nachweisbare Reaktion. Auch andere Arten von Reaktionen sind jedoch grundsätzlich möglich. Insbesondere kann es sich um eine Reaktion handeln, bei welcher bei Anwesenheit des mindestens einen Analyten mindestens ein Nachweisstoff gebildet wird. Dabei können auch mehrere Nachweisstoffe gebildet und/oder verwendet werden, die einzeln, in Gruppen oder alle nachgewiesen werden können. Nachweisstoffe sind also Stoffe, die sich aufgrund der mindestens einen Nachweisreaktion bilden und/oder die an der mindestens einen Nachweisreaktion beteiligt sind und die nachweisbar sind. Anhand des nachgewiesenen mindestens einen Nachweisstoffs kann beispielsweise der mindestens eine Analyt quantitativ und/oder qualitativ nachgewiesen werden. Es sind aber auch Nachweise und/oder Nachweisstoffe möglich, bei denen der Nachweis des mindestens einen Nachweisstoffs nicht oder nicht nur zum Nachweis des Analyten verwendet wird, sondern alternativ beispielsweise zur Bestimmung der Volumenschicht der Probendicke oberhalb des Testfelds, wie im Folgenden noch beschrieben wird.

Die Bildung dieses Nachweisstoffs kann dann direkt oder indirekt nachgewiesen werden. Bei einem direkten Nachweis wird ein Verfahren verwendet, welches unmittelbar die Anwesenheit des Nachweisstoffs qualitativ und/oder quantitativ detektiert. Bei einem indirekten Nachweis hingegen wird über mindestens einen gedanklichen, theoretischen oder experimentellen Zwischenschritt qualitativ und/oder quantitativ auf die Anwesenheit des mindestens einen Nachweisstoffs geschlossen. Beispielsweise kann dies über die Anwesenheit und/oder Bildung und/oder Abnahme von weiteren Stoffen geschehen, wobei beispielsweise aus einer Kenntnis über mindestens einen Reaktionsmechanismus wiederum qualitativ und/oder quantitativ auf den Nachweisstoff geschlossen werden kann, so dass dieser indirekt nachgewiesen werden kann. Ein bekanntestes Beispiel eines derartigen Nachweisstoffs für den Nachweis von Blutglukose ist NADH, also die reduzierte Form von Nicotinamid-Adenin-Dinucleotid (NAD), welches beispielsweise direkt photometrisch nachgewiesen werden kann. Insgesamt kann für die Ausgestaltung möglicher Nachweisreagenzien und Testfelder jedoch weitgehend auf den Stand der Technik verwiesen werden.

Weiterhin umfasst das Testelement mindestens ein Verteilerelement. Dieses Verteilerelement umfasst mindestens eine der Testfeldoberfläche zuweisende Verteileroberfläche, so dass zwischen der Verteileroberfläche und der Testfeldoberfläche mindestens ein Kapillarspalt ausgebildet ist, welcher eine Spaltbreite, also eine Dicke senkrecht zur zugänglichen Testfeldoberfläche aufweist. Der Kapillarspalt ist dabei derart eingerichtet, dass sich innerhalb dieses Kapillarspalts eine Schicht der Probe mit einer Schichtdicke von nicht mehr als 50 $\mu$m ausbilden kann. Besonders bevorzugt ist es, wenn die Schichtdicke nicht mehr als 20 $\mu$m beträgt. Als besonders geeignet haben sich Schichtdicken von 10 $\mu$m oder weniger erwiesen.

Diese Schichtdicke kann auf mehrere Weisen gebildet sein. Bei idealisierten und perfekt glatten Oberflächen, also bei perfekt glatter Testfeldoberfläche und perfekt glatter Verteileroberfläche, würde die maximale Schichtdicke, welche sich ausbilden kann, gerade dem Abstand zwischen diesen Oberflächen entsprechen, also einer Spaltbreite des Kapillarspalts. Diese Spaltbreite lässt sich beispielsweise durch Distanzstücke (Spacer) einstellen. Bei nicht perfekten Oberflächen würden diese Distanzstücke beispielsweise den Abstand zwischen den höchsten Erhebungen der einander gegenüberliegenden Oberflächen vorgeben. Die Spaltbreite kann beispielsweise maximal 50 $\mu$m, insbesondere maximal 30 $\mu$m, vorzugsweise maximal 20 $\mu$m und besonders bevorzugt maximal 10 $\mu$m betragen. In der Regel sind Oberflächenrauigkeiten unvermeidlich oder sogar gewünscht. Diese Oberflächenrauigkeiten führen dazu, dass die Schichtdicke der Probe, also beispielsweise die Flüssigkeits-Schichtdicke einer flüssigen Probe, lokal innerhalb des Kapillarspalts schwanken kann zwischen lokalen Maxima und lokalen Minima. Die angegebenen bevorzugten Schichtdicken beziehen sich dementsprechend auf die maximalen Schichtdicken, welche vorzugsweise nicht überschritten werden. Die Oberflächenrauigkeiten können sogar gezielt genutzt werden. Wie unten exemplarisch näher ausgeführt wird, sollen erfindungsgemäß auch Kapillarspalte mit einer nominellen Spaltbreite "Null" von der Erfindung umfasst sein, also Kapillarspalte, welche dadurch hergestellt werden, dass die Verteileroberfläche unmittelbar auf der Testfeldoberfläche aufliegt. In diesem Fall wird der Kapillarspalt lediglich durch die Oberflächenrauigkeiten der Testfeldoberfläche und/oder der Verteileroberfläche bestimmt beziehungsweise hergestellt. Aufgrund dieser unvermeidlichen oder bewusst erzeugten Unregelmäßigkeiten bildet sich ebenfalls ein Kapillarspalt aus, welcher die unten näher beschriebenen Kapillareffekte bewirkt. Ein derartiges System, bei welchem die Verteileroberfläche unmittelbar auf der Testfeldoberfläche aufliegt, stellt ein "geschlossenes" System dar, bei welchem das Probenvolumen unmittelbar über der Testfeldoberfläche, insbesondere das Probenvolumen pro Flächeneinheit, stark begrenzt ist.

Das Testfeld kann insbesondere mindestens ein Testfeldmaterial aufweisen. Dieses Testfeldmaterial kann vorzugsweise derart ausgestaltet sein, dass dieses eine mittlere Korngröße von weniger als 50 $\mu$m aufweist. Vorzugsweise kann die mittlere Korngröße weniger als 20 Mikrometer betragen, insbesondere weniger als 10 Mikrometer und besonders bevorzugt 5 Mikrometer oder weniger. Der Begriff der Korngröße wird allgemein unten noch näher erläutert.

Unter einem Verteilerelement ist dabei grundsätzlich ein beliebig geformtes Element zu verstehen, welches die der Testfeldoberfläche zuweisende Verteileroberfläche bereitstellt. Sowohl die Testfeldoberfläche als auch die Verteileroberfläche sind dabei vorzugsweise im Wesentlichen glatt und/oder eben ausgestaltet, so dass sich vorzugsweise ein ebener Kapillarspalt ausbildet. Dieser mindestens eine Kapillarspalt kann sich dabei vorzugsweise über die gesamte Testfeldoberfläche hinwegerstrecken. Alternativ kann die Testfeldoberfläche jedoch auch nur teilweise bedeckt sein. Auch die Ausbildung mehrerer einzelner Kapillarspalte ist grundsätzlich möglich. Die Testfeldoberfläche kann beispielsweise einen Auswertebereich umfassen, also einen Bereich, welcher für die Auswertung der Messung, also den Nachweis des mindestens einen Analyten, herangezogen wird. Beispielsweise kann dies, wie unten näher ausgeführt wird, ein Bereich des Testfelds sein, in welchem an dem Trägerelement mindestens ein Nachweisbereich ausgebildet ist. Beispielsweise kann für einen optischen Nachweis des mindestens einen Analyten der Auswertebereich durch den Nachweisbereich hindurch von der dem Testfeld abgewandten Seite des Testelements her optisch zugänglich sein. Der Auswertebereich kann dementsprechend beispielsweise die Projektion des Nachweisbereichs auf die Testfeldoberfläche sein. Vorzugsweise bedeckt der Kapillarspalt den Auswertebereich der Testfeldoberfläche im Wesentlichen vollständig. Auch hier ist jedoch grundsätzlich wiederum eine lediglich unvollständige Bedeckung möglich, beispielsweise indem die Verteileroberfläche lediglich einen Teil des Auswertebereichs bedeckt und/oder indem mehrere Verteileroberflächen oberhalb des Auswertebereichs vorgesehen sind, so dass sich mehrere Kapillarspalte ausbilden.

Das Testelement ist vorzugsweise als flaches, ebenes Testelement ausgestaltet, also als Teststreifen. Vorzugsweise ist dabei die laterale Erstreckung des Teststreifens erheblich größer als die Dicke des Teststreifens, beispielsweise um mindestens einen Faktor 10. Die Testfeldoberfläche kann eine laterale Erstreckung aufweisen, wobei sich der Kapillarspalt vorzugsweise im Wesentlichen gleichförmig und/oder parallel zu dieser lateralen Erstreckung ausdehnt. Unter einer lateralen Erstreckung ist dabei die Erstreckung in Dimensionen senkrecht zur Dicke des Testfelds zu verstehen, wobei die laterale Erstreckung vorzugsweise erheblich größer ist als die Dicke des Testfelds. Die Testfeldoberfläche kann vorzugsweise eben ausgestaltet sein, so dass auch der Kapillarspalt vorzugsweise als ebener Kapillarspalt ausgebildet ist. Auch gekrümmte Testfeldoberflächen sind jedoch grundsätzlich möglich, beispielsweise runde, gewölbte oder auf ähnliche Weise geformte Testfeldoberflächen. Auch in diesem Fall ist vorzugsweise der Kapillarspalt mit einer im Wesentlichen (d.h. beispielsweise über Oberflächenrauigkeiten gemittelten) konstanten Spaltbreite ausgestaltet, so dass die Verteileroberfläche vorzugsweise der Kontur der Testfeldoberfläche folgt. Der Kapillarspalt kann über die gesamte Erstreckung des Kapillarspalts hinweg eine im Wesentlichen konstante Spaltbreite aufweisen, also eine konstante Spaltbreite oder eine Spaltbreite, welche um nicht mehr als 10%, vorzugsweise weniger, von einem Mittelwert abweicht.

Im Folgenden wird begrifflich zwischen der Schichtdicke der Probe, die sich in dem Kapillarspalt einstellen kann, und der Spaltbreite des Kapillarspalts unter Hinweis auf die obige Beschreibung nicht mehr unterschieden. Wenn also von der Spaltbreite die Rede ist, so ist dabei jeweils die Möglichkeit umfasst, dass dies die Schichtdicke der Probe innerhalb des Kapillarspalts ist oder der tatsächliche Abstand zwischen der Testfeldoberfläche und der Verteileroberfläche.

Die Schichtdicke bzw. der Kapillarspalt mit der bevorzugten Dicke von 10 $\mu$m oder weniger, beispielsweise einer Spaltbreite h im Bereich $0 \leq h < 10\ \mu$m, $0 \leq h < 5\ \mu$m oder $5\ \mu$m $\leq h < 10\ \mu$m, hat sich überraschenderweise in der Praxis in verschiedener Hinsicht als äußerst vorteilhaft herausgestellt. So stellt diese Ausgestaltung des Testelements einen Weg bereit, um auf eine kostengünstige Weise, beispielsweise durch lediglich leichte Modifizierung bestehender Systeme, das Spreiten kleiner Probenvolumina, insbesondere flüssiger Proben, von ca. 1 $\mu$l sehr schnell zu bewerkstelligen. Beispielsweise kann eine Ausbreitung (Spreiten) in einem Zeitraum von weniger als 1 s bewerkstelligt werden. Die Probenflüssigkeit kann dabei über eine Platz sparende Fläche von beispielsweise maximal 40 mm$^2$, vorzugsweise maximal 20 mm$^2$, verteilt werden. Beispielsweise können Testfeldoberflächen eingesetzt werden, welche Abmessungen von 5 mm . 4 mm oder weniger aufweisen. Diese schnelle und homogene Verteilung der Probe auf der Testfeldoberfläche wird insbesondere durch Kapillareffekte im Kapillarspalt bewirkt. Dies ist der Kapillareffekt zwischen der Testfeldoberfläche und der Verteileroberfläche, beispielsweise also ein Kapillareffekt zwischen zwei parallelen Platten. Das Testelement kann eingerichtet sein, um Proben mit einem Probenvolumen von weniger als 500 nl aufzunehmen, vorzugsweise von weniger als 300 nl. Dies kann beispielsweise durch eine entsprechende Dimensionierung des Kapillarspalts erfolgen. Dabei bezieht sich das Probenvolumen in dieser bevorzugten Ausführungsform auf das vollständige, von dem Testelement bei einem Nachweis aufgenommene Volumen an Probe. Alternativ oder zusätzlich kann auch das Probenvolumen in dem Kapillarspalt oberhalb des oben beschriebenen Auswertebereichs, also ein Auswertevolumen, sehr klein gehalten werden, beispielsweise bei weniger als 500 nl, vorzugsweise bei weniger als 300 nl und besonders bevorzugt bei weniger als 100 nl. Auch Auswertevolumina von 50 nl und weniger, beispielsweise 10 nl oder weniger, sind möglich.

Das Testelement kann beispielsweise unter Verwendung bekannter Testelemente erzeugt werden, welche lediglich geringfügig durch zusätzliche Aufbringung des mindestens einen Verteilerelements modifiziert werden müssen. Allerdings kann bei dem Testelement vorzugsweise auf ein Spreitnetz auf der Testfeldoberfläche verzichtet werden. Das Testelement kann beispielsweise, wie auch bei herkömmlichen Teststreifen, mindestens ein Trägerelement aufweisen, insbesondere einen Trägerstreifen. Dieses Trägerelement kann beispielsweise ein Kunststoffmaterial, ein Keramikmaterial, ein Papiermaterial, ein Kompositmaterial oder Ähnliches umfassen. Das Testfeld kann dann auf dieses Trägerelement aufgebracht sein. Das Trägerelement kann eingesetzt werden, um eine mechanisch tragende Funktion für das Testelement zu übernehmen, beispielsweise um eine Halterung des Testelements während des Aufbringens der Probe und/oder während einer Messung zu ermöglichen. Der Kapillarspalt kann dann auf der dem Trägerelement gegenüberliegenden Seite des Testfelds angeordnet sein. Dies kann beispielsweise dadurch erfolgen, dass das Verteilerelement mindestens ein Folienelement umfasst, insbesondere mindestens eine Kunststofffolie. Das Folienelement weist vorzugsweise eine geschlossene Oberfläche auf, welche vorzugsweise im Wesentlichen undurchlässig ist gegenüber der Probe. Vorzugsweise ist das Folienelement nicht-porös ausgestaltet, weist eine nichtporöse Oberfläche auf oder weist Poren mit einem mittleren Porenradius von nicht mehr als 5 µm, vorzugsweise von nicht mehr als einem Mikrometer, auf. Damit steht das Folienelement beispielsweise im Gegensatz zu herkömmlichen netzartigen Materialien, beispielsweise Spreitnetzen. Allgemein können das Verteilerelement und/oder die Verteileroberfläche mindestens ein für die Probe im Wesentlichen undurchlässiges Material aufweisen.

Beispielsweise kann dieses Folienelement auf die Testfeldoberfläche aufgebracht sein. Dieses Aufbringen kann beispielsweise derart erfolgen, dass das Trägerelement nur in einem kleinen Bereich, vorzugsweise in einem Bereich, welcher nicht wesentlich über die Testfeldoberfläche hinausragt, durch das Folienelement bedeckt wird. Auf diese Weise bildet sich zwischen dem Verteilerelement und dem Trägerelement der beschriebene Kapillarspalt aus. Zwischen der Testfeldoberfläche und der Verteileroberfläche kann weiterhin mindestens ein Distanzelement angeordnet sein, um eine konstante Dicke des Kapillarspalts, also eine konstante und reproduzierbare Spaltbreite, zu gewährleisten. Wie oben dargestellt, kann der Kapillarspalt jedoch auch durch unmittelbares Aufsetzen der Verteileroberfläche auf die Testfeldoberfläche erzeugt werden.

Das Trägerelement kann ganz oder teilweise aus einem optisch intransparenten Material hergestellt sein. Unter einem optisch intransparenten Material ist dabei ein Material zu verstehen, welches im sichtbaren und/oder infraroten und/oder ultravioletten Spektralbereich eine Transmission von weniger als 5% aufweist. Insbesondere in diesem Fall kann der Nachweis der Reaktion, beispielsweise auf optischem Wege, insbesondere derart erfolgen, dass dieser Nachweis nicht durch das Trägerelement hindurch erfolgt, sondern beispielsweise parallel zur lateralen Erstreckung des Kapillarspalts und/oder senkrecht zu derselben, beispielsweise durch das Verteilerelement hindurch. So kann beispielsweise das Verteilerelement zumindest teilweise optisch transparent ausgestaltet sein. Beispielsweise kann das Verteilerelement, wie oben beschrieben, mindestens ein Folienelement umfassen, beispielsweise eine Folie eines transparenten Kunststoffs. Besonders bevorzugt ist hierbei die Verwendung von Polycarbonat-Folien, beispielsweise von POKALON®-Folien. Grundsätzlich können jedoch auch andere Kunststoffe eingesetzt werden, vorzugsweise optisch transparente Kunststoffe.

Alternativ oder zusätzlich kann der Nachweis der mindestens einen nachweisbaren Reaktion jedoch auch ganz oder teilweise durch das Trägerelement hindurch erfolgen. So kann das Testelement beispielsweise im Bereich des Testfelds auf einer dem Kapillarspalt abgewandten Seite, insbesondere einer dem Kapillarspalt abgewandten Seite des Trägerelements, mindestens einen Nachweisbereich aufweisen, durch welchen hindurch der Nachweis der mindestens einen Reaktion erfolgen kann. So kann die nachweisbare Reaktion beispielsweise mindestens eine optisch nachweisbare Reaktion umfassen, welche von der Seite des Nachweisbereichs her erfolgen kann. In diesem Fall ist es besonders bevorzugt, wenn das Testfeld mindestens ein optisch intransparentes Material, insbesondere ein Reflektormaterial, umfasst, beispielsweise ein Pigment. Beispielsweise können hier Titandioxid-Partikel eingesetzt werden. Dieses optisch intransparente Material kann derart eingerichtet sein, dass der Kapillarspalt auf der Seite des Nachweisbereichs nicht oder nur unwesentlich sichtbar ist. Auf diese Weise kann beispielsweise verhindert werden, dass der optische Nachweis durch die Probe selbst, beispielsweise Hämoglobin der Probe, beeinflusst wird. Diese störenden Probenbestandteile können dann beispielsweise auf der dem Nachweisbereich abgewandten Seite des Testfelds zurückgehalten werden, wie beispielsweise in EP 0 821 233 A2 beschrieben wird. Auf diese Weise lassen sich beispielsweise photometrische Messungen durchführen, welche durch die Probe selbst beeinflusst werden könnten.

Ein optischer Nachweis kann beispielsweise durch eine direkte Lichteinstrahlung und/oder direkte Lichtaufnahme erfolgen. Alternativ oder zusätzlich kann ein optischer Nachweis auch beispielsweise unter Verwendung eines oder mehrerer Lichtleiter erfolgen. So kann wiederum die mindestens eine nachweisbare Reaktion mindestens eine optisch nachweisbare Reaktion umfassen, wobei das Testelement weiterhin mindestens einen Lichtleiter umfasst, welcher eingerichtet ist, um mindestens ein Nachweislicht von dem Testfeld zu einem optischen Detektor zu transportieren. Unter einem Nachweislicht ist dabei ein Licht im infraroten und/oder sichtbaren und/oder ultravioletten Spektralbereich zu verstehen, welches von dem Testfeld emittiert und/oder reflektiert und/oder transmittiert und/oder absorbiert und/oder gestreut wird und welches Informationen über die nachweisbare Reaktion enthalten kann. Beispielsweise kann es sich bei diesem Nachweislicht um ein Lumineszenzlicht, also ein Fluoreszenzlicht und/oder ein Phosphoreszenzlicht, und/oder ein reflektiertes bzw. remittiertes Licht handeln. Optional kann der mindestens eine Lichtleiter weiterhin eingerichtet sein, um mindestens ein Abfragelicht von einer Lichtquelle zu dem Testfeld zu transportieren. Dementsprechend kann der mindestens eine Lichtleiter beispielsweise einen separaten Lichtleiter für das Abfragelicht umfassen und/oder kann einen Lichtleiter in Doppelfunktion für den Transport des Nachweislichts und des Abfragelichts verwenden. Das Abfragelicht kann beispielsweise ein einfaches Beleuchtungslicht umfassen, welches von dem Testfeld reflektiert und/oder transmittiert wird, wobei aus den spektralen Eigenschaften des reflektierten und/oder transmittierten Nachabfragelichts, welches dann als Nachweislicht verwendet wird, beispielsweise auf die mindestens eine nachweisbare Reaktion geschlossen werden kann. Alternativ oder zusätzlich kann das Abfragelicht jedoch auch mindestens ein Anregungslicht umfassen, beispielsweise um eine Lumineszenz in dem Testfeld und/oder in der Probe anzuregen. Verschiedene Ausgestaltungen sind denkbar und werden exemplarisch unten näher beschrieben.

Das Verteilerelement und/oder die Verteileroberfläche sind vorzugsweise derart ausgestaltet, dass diese mindestens ein für die Probe im Wesentlichen undurchlässiges Material aufweisen. So kann das Verteilerelement beispielsweise selbst aus einem derartigen undurchlässigen Material hergestellt sein. Alternativ oder zusätzlich kann jedoch auch mindestens eine Beschichtung vorgesehen sein, welche diese Undurchlässigkeit bewirkt.

Die Verteileroberfläche und vorzugsweise auch die Testfeldoberfläche weisen vorzugsweise hydrophile Eigenschaften auf. Auf diese Weise lässt sich der oben beschriebene Kapillareffekt weiter begünstigen. Diese hydrophilen Eigenschaften können beispielsweise durch entsprechende Materialauswahl und/oder mindestens eine Beschichtung und/oder durch Oberflächenbehandlungen oder Ähnliches herbeigeführt werden. Auf diese Weise lässt sich die Verteilung der Probe in dem Kapillarspalt und auf der Testfeldoberfläche zusätzlich beschleunigen. Aufgrund dieser Kapillareigenschaften, welche für die Verteilung der Probe in dem Kapillarspalt und auf der Testfeldoberfläche sorgen, kann vorzugsweise auf ein Netzwerk oder Spreitnetz, wie es beispielsweise aus der oben beschriebenen EP 0 821 233 A2 bekannt ist, vollständig verzichtet werden.

Das Einbringen der Probe in den Kapillarspalt kann auf verschiedene Weisen erfolgen, welche auch in Kombination eingesetzt werden können. So kann das Testelement beispielsweise mindestens eine Applikationsstelle zum Auftragen der Probe umfassen, welche in Verbindung mit dem Kapillarspalt steht, insbesondere in fluidischer Verbindung. So kann diese Applikationsstelle beispielsweise unmittelbar am Kapillarspalt angeordnet sein, beispielsweise indem ein Bereich des Kapillarspalts eine von außen zugängliche Applikationsöffnung aufweist. Diese Applikationsöffnung kann beispielsweise an einer Stirnseite des Kapillarspalts angeordnet sein. Alternativ oder zusätzlich kann die Applikationsöffnung jedoch auch senkrecht zum Kapillarspalt ausgebildet sein, beispielsweise durch eine entsprechende Aussparung im Verteilerelement selbst und/oder in dem Trägerelement. Beispielsweise kann das Verteilerelement, wie oben dargelegt, ein auf das Trägerelement beziehungsweise auf das Testfeld aufgebrachtes Folienelement umfassen, welches beispielsweise an einer Stirnseite des Testelements eine Aussparung, beispielsweise eine Nut, aufweisen kann, in welche die Probe eingebracht werden kann. Diesbezüglich kann beispielsweise auf die oben dargestellte DE 197 53 847 A1 verwiesen werden.

Alternativ oder zusätzlich zu dieser unmittelbaren fluidischen Verbindung zwischen Applikationsstelle und Kapillarspalt ist auch eine indirekte Verbindung denkbar. So kann beispielsweise das Testelement weiterhin mindestens eine Transportvorrichtung umfassen, welche eingerichtet ist, um die Probe von der Applikationsstelle zu dem Kapillarspalt zu transportieren. Diese Transportvorrichtung kann beispielsweise eine Kapillare umfassen. Beispielsweise kann diese Kapillare wiederum durch einen entsprechenden zweiten Kapillarspalt, welcher jedoch vorzugsweise nicht an das Testfeld angrenzt, gebildet werden. Auch andere Ausgestaltungen sind grundsätzlich möglich. Alternativ oder zusätzlich kann das Testelement auch mindestens ein Lanzettenelement umfassen, insbesondere ein Lanzettenelement mit einer Transportvorrichtung, beispielsweise einer geschlossenen Kapillaren und/oder einem Kapillarspalt, so dass beispielsweise während des Einstichvorgangs bereits eine Probe gesammelt werden kann.

Wie oben dargestellt, weist das Testelement mit dem Kapillarspalt gegenüber bekannten Testelementen eine Vielzahl von Vorteilen auf. So kann beispielsweise ein Verteilerelement in Form einer Folie oberhalb des Testfelds positioniert werden. Eine Messung kann dann beispielsweise durch dieses Folienelement hindurch erfolgen. Auch eine andere Art der Messung, also des Nachweises der mindestens einen nachweisbaren Reaktion, ist jedoch grundsätzlich möglich, beispielsweise eine Messung parallel zum Kapillarspalt und/oder eine Messung durch das Trägerelement hindurch.

Die bevorzugten Spaltbreiten des Kapillarspalts beziehungsweise die bevorzugten Schichtdicken von ca. 10 $\mu$m oder weniger haben sich, wie unten näher ausgeführt wird, in Versuchen als optimal herausgestellt. Zum einen bedingen derartige Kapillarspalte und Spaltbreiten insbesondere bei üblichen flüssigen Proben, beispielsweise wässrigen Proben, beispielsweise Blutproben, nach wie vor noch einen guten Kapillareffekt, der eine schnelle Verteilung der Probe und somit eine rasche Benetzung der Testfeldoberfläche gewährleistet, beispielsweise innerhalb von weniger als 1 s. Zum anderen hat sich jedoch gezeigt, dass sich bei den genannten Spaltbreiten, insbesondere Spaltbreiten von 10 $\mu$m oder weniger, schneller ein Gleichgewichtszustand üblicher nachweisbarer Reaktionen einstellen kann. Diese Theorie, von der die Entdeckung der optimalen Spaltbreiten jedoch grundsätzlich unabhängig ist, basiert beispielsweise auf den oben beschriebenen Reaktionen, bei welchen mindestens ein Nachweisstoff gebildet wird. Dieser Nachweisstoff, beispielsweise NADH, kann beispielsweise in dem Testfeld selbst in gebundener Form vorliegen, beispielsweise in Form einer Komplexbindung. Beispielsweise kann in dem Testfeld ein Komplex vorliegen, in welchem NADH an Glucosedehydrogenase gebunden ist. Weiterhin kann der Nachweisstoff jedoch auch in die Probe selbst hineindiffundieren, beispielsweise in Form von freiem NADH. Je größer das über dem Testfeld zur Verfügung stehende Probenvolumen ist, insbesondere je größer die Spaltbreite ist, desto länger andauernd ist diese Diffusion, da ein bestimmter Konzentrationsgradientenbereich des Nachweisstoffs länger besteht. Mit zunehmendem Probenvolumen, insbesondere mit zunehmender Spaltbreite oberhalb der Testfeldoberfläche, treten also zwei Effekte ein: zum einen wird der Zeitpunkt, zu welchem sich ein Diffusionsgleichgewicht einstellt und keine nennenswerte Diffusion mehr stattfindet, verzögert, so dass eine Messung erst mit erheblich größerer Verzögerung ab Einbringen der Probe in das Testelement zu stabilen und reproduzierbareren Resultaten führt. Zum anderen steht in vielen Fällen der in die Probe hineindiffundierte Nachweisstoff für einen Nachweis nicht mehr zur Verfügung, da viele der herkömmlichen Nachweisverfahren auf dem Nachweis des Nachweisstoffs in dem Testfeld selbst und nicht in der Probe basieren. Beispielsweise basieren übliche, reflektometrische optische Nachweisverfahren, wie oben beschrieben, auf einem rückseitigen Nachweis durch einen Nachweisbereich des Trägerelements hindurch. Dies ist dadurch bedingt, dass bei einem derartigen rückseitigen Nachweis Probenbestandteile, wie beispielsweise Hämoglobin, sich nicht störend auf den optischen Nachweis auswirken können. Diffundiert der Nachweisstoff jedoch in die Probe hinein, so könnte dieser, beispielsweise aufgrund einer begrenzten Eindringtiefe des Anregungslichts und/oder des Beleuchtungsstrahls, für einen derartigen Nachweis nicht mehr zur Verfügung stehen, so dass insgesamt der Signalhub beim Nachweisverfahren zum Nachweis der mindestens einen Reaktion verringert würde.

Es hat sich gezeigt, dass die oben dargestellten bevorzugten Spaltbreiten von 10 $\mu$m oder weniger nicht nur eine schnelle Verteilung kleiner Probenvolumina auf der Testfeldoberfläche bewirken, sondern auch eine schnellere Einstellung des Diffusionsgleichgewichts, bei gleichzeitig noch akzeptablem Signalhub. So kann sich bei einem Kapillarspalt von 10 $\mu$m Spaltbreite oder weniger ein Gleichgewicht der Reaktion bereits innerhalb weniger Sekunden einstellen und kann detektiert werden, was die Messzeit erheblich beschleunigen kann. Wie oben dargelegt, ist dies insbesondere möglicherweise durch die Reaktionskinetik und durch den Diffusionsprozess des Nachweisstoffs bedingt, beispielsweise des NADHs, welches aus dem Testfeld heraus in die Probe, beispielsweise in die auf der Testfeldoberfläche befindliche Flüssigkeitsschicht, diffundiert. Dieser Diffusionsprozess kann durch die begrenzte Spaltbreite verlangsamt und insgesamt in seinem Umfang vermindert werden. Dadurch stellt sich entsprechend schnell ein Diffusionsgleichgewicht zwischen der Probe und dem Testfeld ein.

Der oben beschriebene Effekt einer schnellen Verteilung der Probe auf der Testfeldoberfläche und der beschleunigten Einstellung eines Gleichgewichts ist jedoch von weiteren Faktoren beeinflussbar. So hat es sich herausgestellt, dass insbesondere die Korngröße der in dem Testfeld verwendeten Testfeldmaterialien einen erheblichen Einfluss auf die beschriebenen Effekte haben kann. Die beschriebenen Effekten wurden im Wesentlichen lediglich bei kleinen Korngrößen beobachtet, insbesondere bei Testfeldmaterialien, welche einem Mahlprozess unterworfen worden sind. Dementsprechend wird in einer bevorzugten Ausgestaltung vorgeschlagen, dass das Testfeld mindestens ein Testfeldmaterial umfasst, vorzugsweise vollständig aus diesem Testfeldmaterial zusammengesetzt ist. Unter einem Testfeldmaterial kann dabei auch eine Materialmischung verstanden werden. Dieses Testfeldmaterial kann beispielsweise das oben beschriebene mindestens eine Nachweisreagenz sowie Hilfsstoffe, Füllstoffe, Reflektoren oder Ähnliches umfassen. Zur Herstellung des Testfeldmaterials kann beispielsweise zunächst ein Mahlprozess der einzelnen Komponenten durchgeführt werden, gefolgt von einem Mischprozess, oder umgekehrt. Insbesondere kann bereits eine fertige Materialmischung des Testfeldmaterials einem Mahlprozess, beispielsweise in einer Kugelmühle und/oder einer Jetmühle, unterworfen werden.

Besonders bevorzugt ist es, wenn das Testfeldmaterial, welches in der Regel als partikuläres Material ausgestaltet ist, eine mittlere Korngröße aufweist, welche von 50 nm bis 5 $\mu$m liegt, insbesondere zwischen 50 nm und 5 $\mu$m. Im Gegensatz zu herkömmlichen Testfeldmaterialien, welche Korngrößen im Bereich von mehreren 10 $\mu$m aufweisen können, zeigen derartige Testelemente mit fein gemahlener Testchemie beziehungsweise Testmaterialien die oben beschriebenen positiven Effekte besonders ausgeprägt. Insbesondere lässt sich auf diese Weise die Verteilung der Probe auf der Testfeldoberfläche stark beschleunigen, und auch die Beschleunigung der Einstellung des Gleichgewichts, insbesondere bei den beschriebenen Spaltbreiten von 10 $\mu$m oder weniger, wird deutlich begünstigt. Bei größeren Korngrößen als 5 $\mu$m ist hingegen zum einen eine starke Unregelmäßigkeit zu beobachten, da mit der Korngröße auch die Spaltbreiten zunehmen können. Zudem sinkt die Reproduzierbarkeit der Testelemente, und auch die Benetzungseigenschaften verschlechtern sich. Letzteres kann insbesondere auf eine Vergrößerung des effektiven Kontaktwinkels der Probe auf der Testfeldoberfläche zurückzuführen sein. Bei kleineren Korngrößen als 50 nm hingegen kann ebenfalls die Benetzbarkeit der Testfeldoberfläche deutlich abnehmen. Zudem sind derartig fein gemahlene Testfeldmaterialien technisch nur sehr schwer realisierbar. Unter dem Begriff der Korngröße, wobei hier allgemein die mittlere Korngröße verstanden wird, soll im Rahmen der vorliegenden Erfindung die Größe der einzelnen Partikel des Testfeldmaterials verstanden werden. Diesbezüglich kann beispielsweise auf die entsprechende Norm EN ISO 14688 verwiesen werden. Insbesondere kann unter dem Begriff der Korngröße der Äquivalentdurchmesser verstanden werden, welcher von der hypothetischen Annahme ausgeht, dass die Körner beziehungsweise Partikel des Testfeldmaterials als Kugeln vorliegen. Beispielsweise kann dieser Äquivalentdurchmesser mittels eines Siebdurchmessers angegeben werden. Beispielsweise kann die mittlere Korngröße somit die Korngröße $d_{50}$ sein, also der Median der Korngrößenverteilung. Die mittlere

Korngröße $d_{50}$ ist somit der Äquivalentdurchmesser, der von 50 Massenprozent der Partikel über- bzw. unterschritten wird. Alternativ oder zusätzlich kann auch ein hydrodynamischer Durchmesser als Äquivalentdurchmesser und/oder ein aerodynamischer Durchmesser als Äquivalentdurchmesser verwendet werden. Zur Bestimmung der Korngröße können eine Vielzahl von Verfahren herangezogen werden, beispielsweise Siebverfahren, Sedimentationsverfahren in Wassersäulen, Lichtstreuungsverfahren (beispielsweise Streuung von Laserlicht) oder Ähnliches.

Im Zusammenhang mit der Korngröße steht auch eine bevorzugte Oberflächenrauigkeit der Testfeldoberfläche. So wird vorgeschlagen, dass das Testfeld ein Testfeldmaterial aufweist, wobei das Testfeldmaterial an der Testfeldoberfläche eine Oberflächenrauigkeit von weniger als 50 μm, vorzugsweise von weniger als 25 μm, insbesondere von maximal 5 μm und besonders bevorzugt von maximal 2 μm aufweist. Derartige Oberflächenrauigkeiten lassen sich beispielsweise durch Verwendung der oben beschriebenen Testfeldmaterialien mit den genannten Korngrößen realisieren. Alternativ oder zusätzlich lassen sich jedoch auch geeignete Auftragsverfahren einsetzen, um das Testfeldmaterial zu erzeugen, beispielsweise auf das Trägerelement aufzutragen. Zu diesem Zweck lassen sich beispielsweise Drucktechniken verwenden, insbesondere Siebdruck, Schablonendruck oder Tampondruck. Auch Rakelverfahren lassen sich einsetzen sowie Kombinationen der genannten und/oder anderer Techniken.

Die Verwendung der gemahlenen Testfeldmaterialien mit den beschriebenen Korngrößen führt zudem zu einer verbesserten und homogeneren Benetzung der Testfeldoberfläche. Weiterhin können Grenzflächeneffekte an der Testfeldoberfläche homogenisiert werden, beispielsweise indem eine homogenere Löslichkeit verwendeter Nachweisreagenzien erzielt werden kann. Diese höhere Homogenität macht sich insbesondere wiederum bei der Vermessung von kleinen Probenvolumina positiv bemerkbar, da hierbei eine Mittelung über die Inhomogenitäten der Testfeldoberfläche zur Verfälschung der Messergebnisse führen kann.

Die oben dargestellte Erkenntnis, dass die Einstellung eines stationären Zustands der mindestens einen nachweisbaren Reaktion, insbesondere der in der Regel beteiligten Diffusionsprozesse, von erheblicher Bedeutung ist, kann auch verallgemeinert genutzt werden. So wird ein Testelement zum Nachweis mindestens eines Analyten in einer Probe vorgeschlagen. Insbesondere kann dieses Testelement gemäß einer oder mehrerer der oben dargestellten Ausführungsvarianten ausgestaltet sein. Dementsprechend kann für mögliche Ausgestaltungen, welche einzeln oder in Kombination realisiert werden können, auf die obige Beschreibung verwiesen werden. Auch andere Ausgestaltungen sind jedoch grundsätzlich möglich. Wiederum umfasst das Testelement mindestens ein Testfeld mit einer Testfeldoberfläche, wobei das Testfeld mindestens ein Nachweisreagenz aufweist, welches eingerichtet ist, um bei Anwesenheit des Analyten mindestens eine nachweisbare Reaktion durchzuführen. Das Testelement ist derart eingerichtet, dass sich ein stationärer Zustand der nachweisbaren Reaktion innerhalb einer Zeitdauer von maximal 4 Sekunden ab einem Aufbringen der Probe einstellt, vorzugsweise innerhalb einer Zeitdauer von maximal 3 Sekunden.

Unter einem stationären Zustand ist dabei ein Zustand zu verstehen, in welchem sich die nachweisbaren Parameter der nachweisbaren Reaktion zumindest vorübergehend, beispielsweise für mindestens 1 Sekunde, insbesondere 10 Sekunden, vorzugsweise 1 Minute oder mehr, nur noch unwesentlich ändern. Beispielsweise kann sich ein Plateau in mindestens einem nachweisbaren Parameter einstellen. Bei Remissionsmessungen beispielsweise kann dieses Plateau dadurch definiert sein, dass sich ein relativer Remissionswert innerhalb von einer Sekunde um nicht mehr als um 1,5% relative Remission ändert. Beispielsweise kann dies, wie oben dargestellt, einen stationären Zustand von Diffusionsreaktionen implizieren, beispielsweise einen stationären Zustand, bei welchem sich die Konzentrationsverhältnisse mindestens eines Nachweisstoffs innerhalb des Testfelds und innerhalb der Probe außerhalb des Testfelds nicht mehr oder nur noch unwesentlich ändern. Beispielsweise können sich in dem stationären Zustand Konzentrationsverhältnisse einstellen, bei denen sich die Konzentrationen des mindestens einen Nachweisstoffs um nicht mehr als 10%, insbesondere um nicht mehr als 5% und besonders bevorzugt um nicht mehr als 3% ändern.

Wie oben dargestellt, kann diese Verkürzung der genannten Zeitdauer auf maximal 4 Sekunden beispielsweise durch eine konstruktive Begrenzung der Schichtdicke der Probe oberhalb der Testfeldoberfläche erfolgen, beispielsweise auf eine Schichtdicke von nicht mehr als 50 μm, insbesondere eine Schichtdicke von nicht mehr als 20 μm und besonders bevorzugt auf eine Schichtdicke von 10 μm oder weniger. Diesbezüglich kann wiederum beispielsweise auf die obige Beschreibung verwiesen werden. Beispielsweise kann dies wiederum durch Verwendung eines entsprechenden Kapillarspalts erfolgen. Grundsätzlich sind jedoch auch andere Ausgestaltungen möglich, also Ausgestaltungen ohne die genannte Begrenzung der Schichtdicke und/oder ohne die Verwendung eines Kapillarspalts.

Die oben beschriebene Erkenntnis, dass Diffusionsprozesse das Messergebnis erheblich beeinflussen können, wird zur Verbesserung der Genauigkeit der Messungen verwendet. Dementsprechend werden eine Vorrichtung und ein Verfahren zum Nachweis mindestens eines Analyten in der Probe vorgeschlagen, insbesondere zum Nachweis mindestens eines Metaboliten in einer Körperflüssigkeit. Dabei umfasst die Vorrichtung mindestens ein Testelement, insbesondere ein Testelement nach gemäß einer oder mehreren der oben beschriebenen Ausführungsformen. Dementsprechend kann bezüglich möglicher Ausführungsformen des Testelements auf die obige Beschreibung verwiesen werden. Das Verfahren wird entsprechend unter Verwendung mindestens eines Testelements, insbesondere mindestens eines Testelements gemäß der obigen Beschreibung einer oder mehrerer der beschriebenen Ausführungsformen durchgeführt, so dass dementsprechend ebenfalls auf die obige Beschreibung verwiesen werden kann. Grundsätzlich sind jedoch in der Vorrichtung und/oder in dem Verfahren auch andere Arten von Testelementen einsetzbar, beispielsweise Testelemente ohne Kapillarspalte. Weiterhin kann das Verfahren unter Verwendung einer erfindungsgemäßen Vorrichtung durchgeführt werden.

Das verwendete Testelement weist mindestens ein Testfeld mit einer Testfeldoberfläche zum Aufbringen der Probe auf. Das Testfeld wiederum weist mindestens ein Nachweisreagenz auf, welches eingerichtet ist, um bei Anwesenheit des Analyten eine nachweisbare Reaktion durchzuführen. Dabei ist das Nachweisreagenz derart eingerichtet, dass bei der nachweisbaren Reaktion mindestens ein Nachweisstoff gebildet wird, beispielsweise ein Nachweisstoff gemäß der obigen Beschreibung. Insbesondere kann dieser Nachweisstoff NADH umfassen.

Wie oben dargelegt, spielt insbesondere die Diffusion dieses Nachweisstoffs in die Probe bei vielen Nachweisreaktionen eine entscheidende Rolle. Dementsprechend wird vorgeschlagen, bei dem Verfahren den Nachweisstoff in der Probe außerhalb des Testfelds direkt und/oder indirekt zu bestimmen. Diese Bestimmung kann qualitativ und/oder quantitativ erfolgen, wobei bezüglich der Begriffe "direkt" und "indirekt" auf die obige Definition verwiesen werden kann. Die Bestimmung kann beispielsweise in Form eines direkten Nachweises und/oder in Form eines indirekten Nachweises erfolgen, insbesondere quantitativ. Weiterhin wird vorgeschlagen, dass die Vorrichtung eingerichtet ist, um den Nachweis des Analyten dann unter Berücksichtigung dieser Bestimmung des Nachweisstoffs in der Probe außerhalb des Testfelds durchzuführen. In anderen Worten kann der Analytnachweis derart durchgeführt werden, dass nunmehr auch die bei bisherigen Nachweisverfahren unbekannte Größe der Diffusion in die Probe außerhalb des Testfelds berücksichtigt wird. Dies kann beispielsweise dadurch geschehen, dass Korrekturfaktoren ermittelt werden, welche diese Diffusion berücksichtigen. Alternativ oder zusätzlich können auch andere Arten von Korrektur-Algorithmen angewandt werden, um das Ausmaß der Diffusion des Nachweisstoffs in die Probe außerhalb des Testfelds zu berücksichtigen. So können beispielsweise hinterlegte Korrektur-Algorithmen verwendet werden, beispielsweise Korrekturfaktoren in elektronischen Tabellen, entsprechend des Nachweises des Nachweisstoffs in der Probe außerhalb des Testfelds oder Ähnliches. Mittels der Berücksichtigung des Nachweisstoffs in der Probe außerhalb des Testfelds können beispielsweise Schwankungen in der Flüssigkeitsschichtdicke, insbesondere in der Spaltbreite, beispielsweise aufgrund von herstellungsbedingten Toleranzen in der Testelementproduktion, berücksichtigt werden.

Zur Durchführung dieses Verfahrens kann die Vorrichtung beispielsweise eine Kalibrationsvorrichtung umfassen, welche eingerichtet ist, um den Nachweisstoff in der Probe außerhalb des Testfelds zu bestimmen, beispielsweise direkt, oder indirekt nachzuweisen. Die Vorrichtung kann dann entsprechend eingerichtet sein, um den Nachweis des Analyten unter Berücksichtigung dieser Bestimmung des Nachweisstoffs in der Probe außerhalb des Testfelds durchzuführen. Beispielsweise kann die Vorrichtung, insbesondere die Kalibrationsvorrichtung, zu diesem Zweck ein entsprechendes Datenverarbeitungsgerät umfassen, beispielsweise einen Mikrocomputer, sowie gegebenenfalls optional einen oder mehrere flüchtige und/oder nicht-flüchtige Datenspeicher, beispielsweise zum Speichern einer Liste von Korrekturfaktoren.

Zur Bestimmung insbesondere zum Nachweis des Nachweisstoffs in der Probe außerhalb des Testfelds können dabei verschiedene Verfahren und/oder Vorrichtungen eingesetzt werden. Wie oben dargestellt, kann dabei der Nachweisstoff beispielsweise direkt nachgewiesen werden und/oder auch indirekt, beispielsweise indem die (in der Regel diffusionsbedingte) Abnahme des Nachweisstoffs und/oder einer Verbindung des Nachweisstoffs bzw. einer den Nachweisstoff enthaltenden Substanz innerhalb des Testfelds qualitativ und/oder quantitativ nachgewiesen wird.

Ein Nachweisverfahren kann beispielsweise darauf beruhen, dass der Nachweisstoff in der Probe und in dem Testfeld unterschiedliche Eigenschaften hinsichtlich einer Wechselwirkung mit einem Abfragelicht und/oder hinsichtlich der Aussendung des Nachweislichts aufweist. Beispielsweise können sich aufgrund von Umgebungseffekten spektrale Eigenschaften des Nachweisstoffs in dem Testfeld und in der Probe unterscheiden. Alternativ oder zusätzlich zu derartigen zeitunabhängigen spektralen Effekten können auch zeitliche spektrale Effekte auftreten, welche sich beispielsweise mittels zeitaufgelösten Messungen nachweisen lassen. Diese zeitlichen Effekte, welche den Nachweisstoff in der Probe außerhalb des Testfelds vom Nachweisstoff innerhalb des Testfelds unterscheiden, können sich beispielsweise in unterschiedlichen Lumineszenzlebensdauern, beispielsweise in unterschiedlichen Fluoreszenz-Lebensdauern, auswirken. Beispielsweise wurde beobachtet, dass NADH, welches innerhalb des Testfelds in der Regel in Form eines Glucosedehydrogenase-NADH-Komplexes (GlucDH-NADH) innerhalb des Testfelds eine höhere Fluoreszenz-Lebensdauer aufweist als in freier Form, beispielsweise innerhalb einer wässrigen flüssigen Probe. Es besteht beispielsweise ein Unterschied in den Fluoreszenz-Lebensdauern von nahezu einer Größenordnung, so dass sich durch entsprechende zeitaufgelöste Messungen quantitativ Nachweisstoffe innerhalb der Probe außerhalb des Testfelds und innerhalb des Testfelds unterscheiden lassen. Die Vorrichtung kann dementsprechend eingerichtet sein, um den Nachweisstoff in der Probe außerhalb des Testfelds mittels mindestens einer zeitaufgelösten Messung, insbesondere mittels mindestens einer zeitaufgelösten optischen Messung, insbesondere einer Fluoreszenz-Messung, nachzuweisen.

Wie oben dargestellt, kann diese Bestimmung des Nachweisstoffs in der Probe außerhalb des Testfelds dazu genutzt werden, um eine Kalibration durchzuführen. So kann beispielsweise eine Kalibration auf Toleranzen in der Spaltbreite des Kapillarspalts vorgenommen werden. Dementsprechend können die Vorrichtung und entsprechend auch das vorgeschlagene Verfahren insbesondere derart ausgestaltet sein, dass das Testelement mindestens einen mit dem Testfeld in Verbindung stehenden Kapillarspalt zum Aufnehmen und Verteilen der Probe auf dem Testfeld umfasst. Beispielsweise kann der Kapillarspalt gemäß einer oder mehrerer der oben beschriebenen Ausführungsformen ausgestaltet sein.

Die Kalibrationsvorrichtung kann dann derart eingerichtet sein, dass mittels der Bestimmung des Nachweisstoffs in der Probe außerhalb des Testfelds einer Kalibration einer Spaltbreite des Kapillarspalts vorgenommen werden kann. Auch dies kann beispielsweise wiederum mittels entsprechender Korrekturfaktoren erfolgen. Durch diese Ausgestaltung des vorgeschlagenen Verfahrens und der vorgeschlagenen Vorrichtung lassen sich insgesamt die Genauigkeiten des Nachweises des mindestens einen Analyten erheblich verbessern, da der Nachweis somit zumindest weitgehend unabhängig von den Probenvolumina ausgestaltet werden kann. Es kann insbesondere eine Online-Kalibration erfolgen, welche gleichzeitig oder lediglich mit geringem Zeitversatz (beispielsweise von weniger als 1 s) zur eigentlichen Messung durchgeführt wird, so dass beispielsweise auf die Eingabe chargenspezifischer Informationen über das mindestens eine Testelement in die Vorrichtung verzichtet werden kann. Die Vorrichtung kann dementsprechend eingerichtet sein, um unmittelbar bei, unmittelbar vor oder unmittelbar nach der eigentlichen Messung eine Kalibrationsmessung durchzuführen, mittels derer die benötigten Kalibrationsinformationen gewonnen werden können. Hierdurch lassen sich beispielsweise die Herstellungsverfahren der Testelemente stark vereinfachen, da geringere Anforderungen an die Toleranzen der Testelemente zu stellen sind.

Ausführungsbeispiele

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigt:

Figur 1   ein Ausführungsbeispiel einer erfindungsgemäßen Vorrichtung zum Nachweis eines Analyten in einer Körperflüssigkeit mit einem ersten Ausführungsbeispiel eines Testelements;

Figuren 2A und 2B   verschiedene Ansichten eines zweiten Ausführungsbeispiels eines Testelements;

Figur 3   ein drittes Ausführungsbeispiel eines Testelements;

Figuren 4A und 4B   ein Beispiel einer Benetzung eines Kapillarspalts mit Wasser;

Figuren 5A und 5B   Beispiele der Benetzung einer Testfeldoberfläche mit einem ungemahlenen Testfeldmaterial und einem gemahlenen Testfeldmaterial;

Figuren 6A und 6B   Beispiele eines zeitlichen Verlaufs einer photometrischen Messung an Testelementen mit gemahlenen und ungemahlenen Testfeldmaterialien;

Figur 7   eine Veranschaulichung von Diffusionseffekten an einer Testfeldoberfläche;

Figur 8   eine zeitliche Entwicklung einer Fluoreszenz von freiem und gebundenem NADH;

Figuren 9A bis 9C   Querschnittsaufnahmen eines Testelements zur Verdeutlichung einer Trennung der Lumineszenz von freiem und gebundenem NADH; und

Figur 10   Grauwertänderungen als Funktion der Zeit bei Testelementen mit verschiedenen Spaltbreiten.

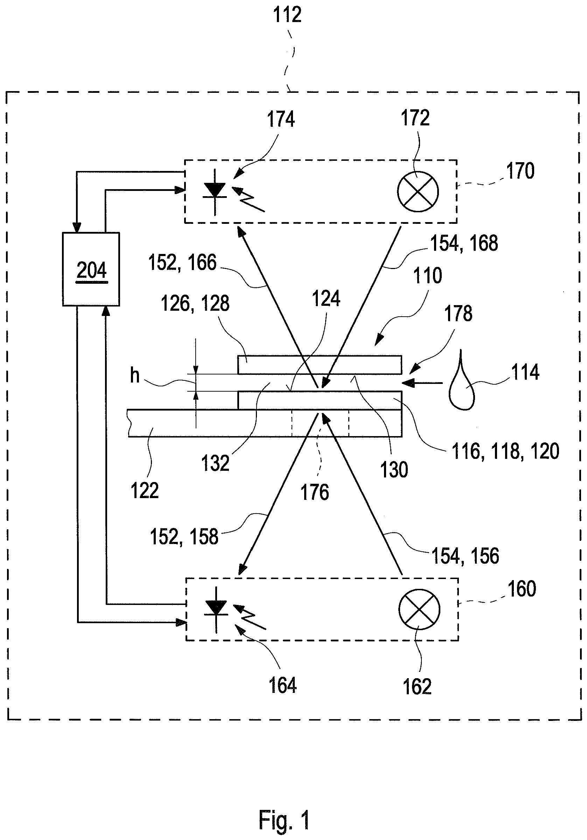

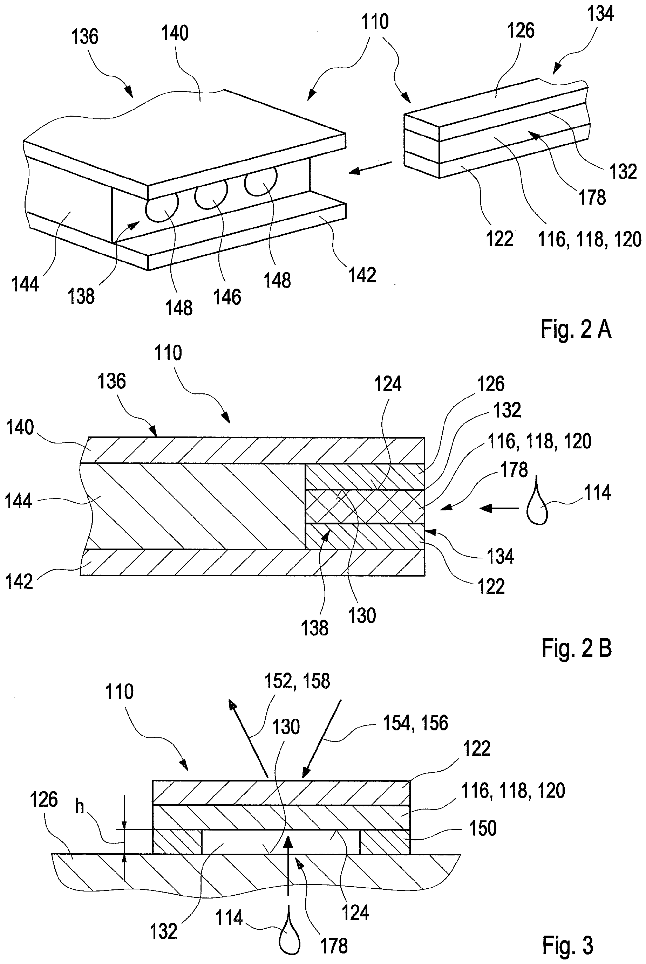

In den Figuren 1, 2A und 2B und in Figur 3 sind drei verschiedene Ausführungsbeispiele erfindungsgemäßer Testelemente 110 zum Nachweis eines Analyten in einer Probe dargestellt. Figur 1 zeigt darüber hinaus symbolisch eine Vorrichtung 112 zum Nachweis des Analyten in einer Körperflüssigkeit, welche das Testelement 110 verwendet. Am Beispiel dieser Vorrichtung 112 soll auch ein erfindungsgemäßes Verfahren zum Nachweis des Analyten in der Probe dargestellt werden.

Im Weiteren sei angenommen, dass es sich bei der Probe um eine Probe einer Körperflüssigkeit handelt, insbesondere eine Blutprobe. Diese Blutprobe ist in Figur 1 und Figur 2B symbolisch durch einen Blutstropfen dargestellt und mit der Bezugsziffer 114 bezeichnet, und es wird angenommen, dass als Analyt beispielsweise Blutglucose in diesem Blutstropfen 114 nachgewiesen werden soll. Es sei jedoch, wie oben ausgeführt, darauf hingewiesen, dass auch andere Arten von Proben untersucht werden können und dass auch ein Nachweis anderer Arten von Analyten erfolgen kann.

Die Testelemente 110 weisen jeweils ein Testfeld 116 auf. Dieses Testfeld 116 umfasst ein Testfeldmaterial 118 mit mindestens einem Nachweisreagenz 120. Dieses Nachweisreagenz 120 ist eingerichtet, um eine Analyt-spezifische, nachweisbare Reaktion durchzuführen, wenn dieses in Kontakt mit dem nachzuweisenden Analyten gerät. Beispielsweise kann dieses Nachweisreagenz 120, wie oben ausgeführt und wie beispielsweise im Stand der Technik beschrieben, ein oder mehrere Enzyme, Hilfsstoffe, Fluorphore oder Ähnliches umfassen. Für den Nachweis von Blutglucose kann beispielsweise das Nachweisreagenz 120 Glucosedehydrogenase als Enzym und NAD+ als Coenzym umfassen. Bei Anwesenheit von Glucose bildet sich dabei NADH, welches als Nachweisstoff dienen kann und welches in freier oder gebundener Form beispielsweise durch photometrische Messungen und/oder durch Lumineszenzmessungen nachgewiesen werden kann, wie unten näher ausgeführt wird.

Das Testfeld 116 ist in dem Ausführungsbeispiel gemäß Figur 1 auf ein Trägerelement 122 aufgebracht. Dieses Trägerelement 122 kann beispielsweise als streifenförmiges Testträgerelement 122 ausgestaltet sein, wobei, wie oben ausgeführt, auch andere Ausführungsformen möglich sind. Das Testfeld 116 weist eine von dem Trägerelement 122 wegweisende Testfeldoberfläche 124 auf. Im Folgenden sei angenommen, dass diese Testfeldoberfläche 124 als ebene Oberfläche ausgestaltet ist, beispielsweise als ebene Testfeldoberfläche mit Abmessungen von 5 . 4 mm$^2$.

Oberhalb der Testfeldoberfläche 124 ist in dem Ausführungsbeispiel gemäß Figur 1 ein Verteilerelement 126 angeordnet, welches in diesem Ausführungsbeispiel als Folienelement 128 ausgebildet ist und welches vorzugsweise transparent ausgestaltet ist. Dieses Verteilerelement 126 weist eine der Testfeldoberfläche 124 zuweisende Verteileroberfläche 130 auf. Auch diese Verteileroberfläche 130 ist vorzugsweise eben ausgestaltet und ist vorzugsweise parallel zur Testfeldoberfläche 124 angeordnet. Zwischen der Verteileroberfläche 130 und der Testfeldoberfläche 124 bildet sich damit ein Kapillarspalt 132 aus, vorzugsweise ein ebener Kapillarspalt 132, welcher sich senkrecht zur Zeichenebene in Figur 1 und damit parallel zur Testfeldoberfläche 124 und deren lateraler Erstreckung ausdehnt. Dieser Kapillarspalt 132 weist eine Spaltbreite auf, welche in Figur 1 mit h bezeichnet ist. Diese Spaltbreite kann beispielsweise, wenn Oberflächenrauigkeiten der Testfeldoberfläche 124 und/oder der Verteileroberfläche 130 vernachlässigt werden und/oder vernachlässigbar sind, im Wesentlichen einer Schichtdicke einer Probe, insbesondere einer flüssigen Probe entsprechen, welche in diesen Kapillarspalt 132 eingebracht wird. Im Folgenden wird daher zwischen der Spaltbreite des Kapillarspalts 132 und der Schichtdicke der Probe innerhalb des Kapillarspalts 132 begrifflich nicht unterschieden, sofern nicht ausdrücklich darauf hingewiesen wird.

In den Figuren 2A und 2B ist ein zweites Ausführungsbeispiel eines Testelements 110 dargestellt. Dabei zeigt Figur 2A eine perspektivische Darstellung, wohingegen Figur 2B eine Schnittdarstellung von der Seite zeigt. Wiederum weist das Testelement 110 gemäß diesem Ausführungsbeispiel ein Testfeld 116 auf, welches auf ein Trägerelement 122 aufgebracht ist. Für mögliche Details kann auf die Beschreibung der Figur 1 verwiesen werden. Dieses Testfeld 116 weist wiederum eine Testfeldoberfläche 124 auf, welche vom Trägerelement 122 weg weist. Entsprechend ist wiederum auf der dem Trägerelement 122 gegenüberliegenden Seite ein Verteilerelement 126 mit einer Verteileroberfläche 130 ausgebildet. Zwischen der Verteileroberfläche 130 und der Testfeldoberfläche 124 bildet sich wiederum ein in Figur 2B im Vergleich zur Schichtdicke des Testfelds 116 sehr klein dimensionierter Kapillarspalt 132 aus. Wie oben beschrieben, kann die Verteileroberfläche 130 auch direkt auf der Testfeldoberfläche 124 aufliegen, so dass sich ein geschlossenes System mit der Spaltbreite "Null" ausbildet, wie oben beschrieben.

Im Gegensatz zum Ausführungsbeispiel gemäß Figur 1, in welchem das Trägerelement 122 erheblich größer dimensioniert ist als das Verteilerelement 126, sind in dem Ausführungsbeispiel gemäß den Figuren 2A und 2B das Trägerelement 122 und das Verteilerelement 126 näherungsweise gleich dimensioniert. Dabei können für das Trägerelement 122 und für das Verteilerelement 126 gleiche oder unterschiedliche Materialien verwendet werden, beispielsweise jeweils optisch transparente oder auch optisch nichttransparente Materialien. Besonders bevorzugt ist die Verwendung von Kunststoff-Folienmaterialien, wie beispielsweise Polycarbonaten, insbesondere POKALON®. Das Trägerelement 122, das Verteilerelement 126 und das dazwischenliegende Testfeld 116 sowie der Kapillarspalt 132 bilden in dem Ausführungsbeispiel gemäß den Figuren 2A und 2B gemeinsam ein Nachweisteil 134, welches beispielsweise sehr klein dimensioniert werden kann, beispielsweise mit Abmessungen von 300 $\mu$m . 300 $\mu$m· 4 mm. Weiterhin kann das Testelement 110 ein Koppelteil 136 umfassen, welches der mechanischen Halterung des Nachweisteils 134 dienen kann. Dieses Koppelteil 136 kann, beispielsweise an seinem vorderen Ende, eine Aufnahme 138 aufweisen, in welche das Nachweisteil 134 eingeschoben werden kann. Diese Aufnahme 138 kann beispielsweise dadurch gebildet werden, dass das Koppelteil 136 einen Sandwich-Aufbau mit einem oberen Haltestück 140, einem unteren Haltestück 142 und einem dazwischenliegenden Koppelkörper 144 aufweist. Wie insbesondere aus Figur 2B erkennbar ist, ragen die Haltestücke 140, 142 im Bereich der Aufnahme 138 über den Koppelkörper 144 hinaus, so dass sich in diesem Bereich die Aufnahme 138 ausbildet, in welche das Nachweisteil 134 eingeschoben werden kann.

Die Haltestücke 140, 142 können beispielsweise wiederum streifenförmig ausgebildet sein, beispielsweise aus einem Kunststoff-, Papier-, Keramik- oder Kompositmaterial. Auch ein komplexerer Schichtaufbau ist möglich. In dem Koppelkörper 144, welcher beispielsweise als ein Kunststoffmaterial umfassen kann, sind, wie in Figur 2A erkennbar, vorzugsweise Lichtleiter 146, 148 eingebettet, welche im Bereich der Aufnahme 138 münden und welche eine optische Ankopplung an das Nachweisteil 134 ermöglichen. In dem dargestellten Ausführungsbeispiel ist ein zentraler Lichtleiter 146 vorgesehen, welcher Abfragelicht von einer in den Figuren 2A und 2B nicht dargestellten Lichtquelle, beispielsweise einer Lichtquelle der Vorrichtung 112, zu dem Nachweisteil 134 und dort insbesondere zu dem Testfeld 116 leiten kann. Dieser zentrale Lichtleiter 146 ist in dem dargestellten Ausführungsbeispiel symmetrisch von zwei weiteren Lichtleitern 148 umgeben, welche eingerichtet sind, um Nachweislicht von dem Nachweisteil 134 zu einem Detektor zu führen, welcher in den Figuren 2A und 2B ebenfalls nicht dargestellt ist, beispielsweise einem Detektor der Vorrichtung 112. Verschiedene Messprinzipien werden unten näher erläutert. Der Koppelkörper 144 dient somit in dem dargestellten Ausführungsbeispiel nicht nur als Distanzstück zwischen den Haltestücken 140, 142 und zur Ausbildung einer mechanischen Aufnahme 138, sondern gleichzeitig zur Bereitstellung einer optischen Kopplung durch die Lichtleiter 146, 148.

Die Fixierung des Nachweisteils 134 in der Aufnahme 138 kann dabei beispielsweise formschlüssig, kraftschlüssig oder stoffschlüssig sowie durch Kombinationen der genannten Verfahren erfolgen. Beispielsweise kann ein Klebstoff zur Fixierung des Nachweisteils 134 in der Aufnahme 138 verwendet werden. Für viele Anwendungen ist jedoch die Verwendung eines Klebstoffs unerwünscht, da dieser beispielsweise selbst Fluoreszenzeigenschaften aufweisen kann.

Die dargestellte optische Kopplung, welche in dem Ausführungsbeispiel gemäß den Figuren 2A und 2B im Wesentlichen parallel zur lateralen Erstreckung des Testfelds 116 erfolgen kann, kann dadurch erleichtert werden, dass die Dicke des Testfelds 116 angepasst werden kann, um die Enden der Lichtleiter 146, 148, beispielsweise die Faserenden, entsprechend zu positionieren. Eine vertikale Orientierung der Enden der Lichtleiter 146, 148 kann dementsprechend beispielsweise allein durch die Foliendicke des Schichtaufbaus des Nachweisteils 134 eingestellt werden.

Weiterhin können bei dem Schichtaufbau, bei welchem eine Kopplung im Wesentlichen parallel zur lateralen Erstreckung des Testfelds 116 erfolgt, für das Trägerelement 122 und/oder das Verteilerelement 126 auch optisch in transparente Materialien, beispielsweise schwarze Materialien, beispielsweise schwarze Folien, verwendet werden. Auf diese Weise kann beispielsweise eine Einkopplung von störendem Umgebungslicht in die Lichtleiter 146, 148 vermieden werden. Alternativ oder zusätzlich können auch das obere Haltestück 140 und/oder das untere Haltestück 142 optisch intransparent sein, beispielsweise wiederum schwarze Folien umfassen, um ebenfalls hier eine Einkopplung störenden Umgebungslichts in die Lichtleiter 146, 148 zu vermeiden und/oder zu reduzieren.

Die Lichtleiter 146, 148 können beispielsweise Glasfasern und/oder Kunststofffasern umfassen. Auch andere Arten von Lichtleitern sind jedoch grundsätzlich einsetzbar, beispielsweise Lichtleiter mit einer anderen Geometrie. Beispielsweise lassen sich derartige Lichtleiter 146, 148 durch Mikro-Spritzguss auf einfache Weise realisieren. Auch andere Techniken sind jedoch einsetzbar, beispielsweise ein Umspritzen von Lichtleiterfasern 146, 148 mit einem Matrixmaterial des Koppelkörpers 144.

In Figur 3 ist ein drittes Ausführungsbeispiel eines Testelements 110 dargestellt. Wiederum weist dieses Testelement 110 ein Testfeld 116 mit einer Testfeldoberfläche 124 auf, welches auf einem Trägerelement 122 aufgebracht ist. Für die Ausgestaltung dieses Testfelds 116 kann beispielsweise wiederum auf die Beschreibung der Figur 1 verwiesen werden. Weiterhin weist das Testelement 110 wiederum ein der Testfeldoberfläche 124 gegenüberliegendes Verteilerelement 126 auf, mit einer der Testfeldoberfläche 124 gegenüberliegenden Verteileroberfläche 130. Zwischen dem Testfeld 116 und/oder dem Trägerelement 122 außerhalb des Testfelds 116 und dem Verteilerelement 126 können, wie in Figur 3 dargestellt und wie auch optional in den Ausführungsbeispielen gemäß den Figuren 1 und 2A und 2B realisierbar, ein oder mehrere Distanzelemente 150 vorgesehen sein. Diese Distanzelemente 150, welche beispielsweise wiederum aus einem Folienmaterial hergestellt sein können, beispielsweise ausgeschnitten oder ausgestanzt sein können, können zur Erzeugung eines konstanten Kapillarspalts 132 mit exakt definierter Spaltbreite h verwendet werden. Vorzugsweise ist dieses mindestens eine Distanzelement 150 derart ausgestaltet, dass der Kapillarspalt 132 an mindestens zwei Seiten geöffnet ist, um einen Druckausgleich zu ermöglichen und um eine Kapillarwirkung zu begünstigen.

Das Ausführungsbeispiel des Testelements 110 gemäß Figur 3 stellt in gewisser Weise einen inversen Aufbau des Ausführungsbeispiels gemäß Figur 1 dar, da in dem dargestellten Ausführungsbeispiel das Trägerelement 122 erheblich kleiner dimensioniert ist als das Verteilerelement 126. Dem Verteilerelement 126 kann somit in dem in Figur 3 dargestellten Ausführungsbeispiel mechanisch tragende Funktion zukommen.

Weiterhin zeigen die Ausführungsbeispiele in den Figuren 1, 2A und 2B und in Figur 3 auch verschiedene Formen des Nachweises der Analyt-spezifischen Reaktion. In allen Fällen ist ein optischer Nachweis dargestellt, welcher im Rahmen der vorliegenden Erfindung besonders bevorzugt ist. Alternativ oder zusätzlich könnten jedoch grundsätzlich auch andere Nachweisverfahren eingesetzt werden, beispielsweise elektrochemische Nachweisverfahren. Bei den dargestellten optischen Nachweisverfahren wird mindestens ein von dem Testfeld 116 ausgehendes Nachweislicht von einem Detektor empfangen. Verschiedene Arten von Nachweislicht wurden oben erläutert. So kann dieses Nachweislicht beispielsweise ein von dem Testfeld 116 reflektiertes, transmittiertes oder emittiertes Licht umfassen und/oder Kombinationen der genannten Lichtarten. Unter der obigen Begriffswahl, dass dieses Nachweislicht von dem Testfeld 116 ausgeht, ist, neben einem Ausgang unmittelbar von der Testfeldoberfläche 124 und/oder dem Inneren des Testfelds 116, auch die Möglichkeit umfasst, dass dieses Nachweislicht von der Probe 114 im Inneren des Kapillarspalts 132 ausgeht. Diese Möglichkeit wird unten näher erläutert.

Das Nachweislicht ist in den Figuren 1 und 3 symbolisch mit der Bezugsziffer 152 gekennzeichnet. Je nach Art und Weise, wie dieses Nachweislicht 152 erzeugt wird, welches die Analyt-spezifische Reaktion nachweisen soll, kann es erforderlich sein, das Testfeld 116 mit einem Abfragelicht 154 zu bestrahlen. Dieses Abfragelicht 154 kann auf unterschiedliche Weise mit dem Testfeld 116 wechselwirken, was, wie oben dargestellt, auch eine Wechselwirkung mit der Probe 114 im Kapillarspalt 132 über der Testfeldoberfläche 124 umfassen soll. Beispielsweise kann dieses Abfragelicht 154 von dem Testfeldmaterial 118 des Testfelds 116 reflektiert und/oder remittiert und/oder absorbiert und/oder gestreut werden, was beispielsweise für photometrische Messungen in üblichen Teststreifen ausgenutzt wird. Beispielsweise kann diese Remission eine Farbänderung des Testfeldmaterials 118 nachweisen, welche durch die Analyt-spezifische Reaktion bedingt ist, beispielsweise durch die Bildung von freiem oder gebundenem NADH als Nachweisstoff. Dementsprechend kann das Abfragelicht 154 beispielsweise einen Beleuchtungsstrahl 156 enthalten, und das Nachweislicht 152 kann einen Remissionsstrahl 158 umfassen. Die Vorrichtung 112 kann dementsprechend, wie in Figur 1 dargestellt, eine photometrische Messvorrichtung 160 mit einer Lichtquelle 162 zur Erzeugung des Beleuchtungsstrahls 156 sowie mit einem Detektor 164 zum Nachweis des Remissionsstrahls 158 umfassen. Die Lichtquelle 162 und der Detektor 164 können dabei auf verschiedene Weisen ausgestaltet sein, beispielsweise unter Verwendung von Lampen, Leuchtdioden, Lasern oder Kombinationen der genannten und/oder anderer Arten von Lichtquellen und unter Verwendung von Photodioden, Photozellen, Photoverstärker oder anderen Arten von Detektoren.

Weiterhin kann, alternativ oder zusätzlich, das Nachweislicht 152 beispielsweise ein Lumineszenzlicht 166 umfassen. Diese Option, welche auch in den anderen Ausführungsbeispielen realisierbar ist, ist exemplarisch in Figur 1 gezeigt. Beispielsweise kann dieses Lumineszenzlicht 166 ein Fluoreszenzlicht sein, welches von einem bei der Analyt-spezifischen Reaktion gebildeten Nachweisstoff emittiert wird. Beispielsweise kann dies wiederum NADH in freier oder gebundener Form sein, wie unten näher erläutert wird. Zur Anregung der Lumineszenz kann das optionale Abfragelicht 154 einen Anregungsstrahl 168 enthalten, wie ebenfalls in Figur 1 symbolisch angedeutet ist. Dieser Anregungsstrahl 168 kann beispielsweise, wie auch das Abfragelicht 154 allgemein, auf die spektralen Eigenschaften des nachzuweisenden Nachweisstoffs angepasst sein. Beispielsweise kann, wie in Figur 1 angedeutet, die Vorrichtung 112 eine Lumineszenz-Messvorrichtung 170 umfassen, mit einer Lichtquelle 172 und einem Detektor 174, welche in Figur 1 wiederum symbolisch angedeutet sind. Für die möglichen Ausgestaltungen der Lichtquelle 172 und des Detektors 174 kann beispielsweise auf die photometrische Messvorrichtung 160 verwiesen werden. So, wie die photometrische Messvorrichtung 160 mit ihrer Lichtquelle 162 und ihrem Detektor 164 speziell auf eine Remissionsmessung angepasst sein kann, kann die Lumineszenz-Messvorrichtung 170 mit ihrer Lichtquelle 172 und ihrem Detektor 174 speziell auf den Lumineszenznachweis angepasst sein. Allgemein kann dabei auch Nachweislicht 152 mit mehreren Wellenlängen empfangen werden. Je nach Art des Nachweislichts 152 kann grundsätzlich auch auf ein Abfragelicht 154 verzichtet werden.

Die Ausführungsbeispiele in den einzelnen Figuren zeigen dabei, dass die Detektion des Nachweislichts 152 auf unterschiedliche Weisen erfolgen kann, welche auch kombiniert einsetzbar sind. Wie in den Figuren 1 und 3 erkennbar, kann das Nachweislicht 152 beispielsweise durch das Trägerelement 122 hindurch detektiert werden, wobei die Vorrichtung 112 beispielsweise entsprechend auf der dem Testfeld 116 gegenüberliegenden Seite des Trägerelements 122 angeordnet sein kann. Dementsprechend kann das Trägerelement 122 beispielsweise, wie in Figur 3 bevorzugt, zumindest teilweise für das Nachweislicht 152 transparent ausgestaltet sein, beispielsweise indem für dieses Trägerelement 122 eine transparente Folie verwendet wird, beispielsweise POKALON®. Alternativ oder zusätzlich kann, wie in Figur 1 gezeigt, das Trägerelement 122 im Bereich des Testfelds 116 auch einen Nachweisbereich 176 umfassen, welcher zumindest teilweise für das Nachweislicht 152 transparent ausgestaltet sein kann. Dieser Nachweisbereich 176 kann beispielsweise eine Öffnung im Trägerelement 122 umfassen und/oder ein optisch transparentes Material.

Wie in den Figuren 1 und 3 dargestellt, ist die rückseitige Messmethode, bei welcher durch das Trägerelement 122 hindurch das Nachweislicht 152 empfangen wird und, optional, auch das Abfragelicht 154 den das Trägerelement 122 durchdringt, besonders bevorzugt für photometrische Messungen, insbesondere Remissionsmessungen. Dabei hat es sich jedoch, insbesondere bei Blutproben, gezeigt, dass derartige photometrische Messungen durch die Probe 114 selbst beeinflusst werden können, da beispielsweise Hämoglobin derartige photometrische Messungen erheblich beeinflussen kann. Dementsprechend hat es sich, wie auch aus dem Stand der Technik bekannt, als vorteilhaft erwiesen, wenn das Testfeldmaterial 118 mindestens ein Pigment umfasst, welches als Reflektormaterial dient und welches zumindest in oberen, dem Kapillarspalt 132 zuweisenden Schichten des Testfelds 116 angeordnet ist. Beispielsweise können zu diesem Zweck Titandioxid-Pigmente verwendet werden. Beispielsweise kann das Testfeld 116 einen Schichtaufbau aufweisen, wie beispielsweise aus dem oben dargestellten Stand der Technik bekannt ist. Durch diese Pigmente können rote Blutkörperchen im oberen, vom Trägerelement 122 abgewandten Bereich des Testfelds 116 zurückgehalten werden, um auf der dem Trägerelement 122 zuweisenden Seite die photometrische Messungen nicht oder nur in geringerem Maße zu beeinflussen. Gleichzeitig dienen die Pigmente als Reflektormaterial, um den rückseitig eingestrahlten Beleuchtungsstrahl 156 zu reflektieren. Die roten Blutkörperchen auf Seiten des Kapillarspalts 132 sind dann vorzugsweise rückseitig, das heißt vom Trägerelement 122 her, nicht sichtbar.

Alternativ oder zusätzlich kann eine in Figur 1 gezeigte Messmethode eingesetzt werden, welche auch in den anderen Ausführungsbeispielen realisierbar ist. Bei dieser Messmethode, welche, wie oben dargestellt, den Nachweis von Lumineszenzlicht 166 als Nachweislicht 152 beinhaltet, kann auch eine Messung durch das Verteilerelement 126 hindurch erfolgen, also von der "Vorderseite" her, mit Blick auf die Testfeldoberfläche 124. Zu diesem Zweck kann das Verteilerelement 126 ganz oder teilweise optisch transparent für das Abfragelicht 154 und/oder Teile des Abfragelichts 154 ausgestaltet sein, beispielsweise wiederum durch Verwendung eines Folienelements 128, beispielsweise einer POKALON®-Folie. Wie unten noch näher erläutert wird, ist diese Art des Nachweises besonders günstig für Lumineszenzlicht-Messungen.

Wiederum alternativ oder zusätzlich kann die in den Figuren 2A und 2B angedeutete Messmethode verwendet werden, bei welcher das Nachweisteil 134 derart in die Aufnahme 138 eingefügt wird, dass die optische Ein- und Auskopplung parallel zur lateralen Erstreckung des Testfelds 116 erfolgen kann. Auch diese Art der Kopplung kann photometrische Messungen und/oder Lumineszenz-Messungen beinhalten, beispielsweise analog zu den oben beschriebenen Messmethoden. Es sei darauf hingewiesen, dass jedoch auch die in den Figuren 2A und 2B dargestellte Ausführungsform derart modifiziert werden kann, dass eine optische Kopplung aus anderen Richtungen erfolgen kann, beispielsweise wiederum senkrecht zur lateralen Erstreckung des Testfelds 116. Zu diesem Zweck kann beispielsweise das Nachweisteil 134 in Figur 2B um 90° oder um andere Winkel um eine Achse senkrecht zur Zeichenebene gedreht werden.

Die Testelemente 110 weisen vorzugsweise eine Applikationsstelle 178 auf, welche in den dargestellten Ausführungsbeispielen als einfache Öffnung des Kapillarspalts 132 zur Umgebung ausgestaltet ist. Die Applikationsstelle 178 kann jedoch auch auf komplexere Weise ausgestaltet sein, beispielsweise durch Verwendung entsprechender Nuten, Öffnungen oder Ähnlichem, beispielsweise im Trägerelement 122 und/oder im Verteilerelement 126. Verschiedene Ausgestaltungen sind möglich.

Erfindungsgemäß wird der Kapillarspalt 132 genutzt, um eine kostengünstige und effiziente Weise bereitzustellen, um auch kleine Probenvolumina von beispielsweise 1 µl innerhalb kurzer Zeit (beispielsweise innerhalb von weniger als 1 s) homogen über die Testfeldoberfläche 124 zu verteilen (Spreiten). Dazu wird der Kapillareffekt zwischen der Verteileroberfläche 130 und der Testfeldoberfläche 124 ausgenutzt, indem beispielsweise über das Testfeld 116 (Reaktionsschicht) eines herkömmlichen Glucoseteststreifens das Verteilerelement 126 aufgebracht wird. Die Testfeldoberfläche 124 und/oder die Verteileroberfläche 130 können dabei, um den Kapillareffekt zu steigern, zusätzlich mit hydrophilen Eigenschaften ausgestattet werden. So kann das Verteilerelement 126 beispielsweise eine hydrophile Folie sein und/oder eine derartige Folie umfassen, beispielsweise das oben bereits erwähnte durchsichtige POKALON®. Auf diese Weise kann ein Sandwich-System entstehen, welches beispielsweise von einer Applikationsstelle 178, beispielsweise am Rand des Testfelds 116, her benetzt wird. So kann über den Kapillareffekt der beiden Oberflächen 124, 130 die Probe 114, beispielsweise Blut, über die an sich in der Regel schlecht zu benetzende Testfeldoberfläche 124 befördert und verteilt werden.

Wie oben dargestellt, hat es sich dabei überraschenderweise als vorteilhaft erwiesen, wenn sehr kleine Kapillarspalte 132 verwendet werden, also Kapillarspalte 132 mit einer sehr geringen Spaltbreite h. Diese Spaltbreite h sollte bei 10 µm oder weniger liegen. Dies kann beispielsweise, wie in Figur 3 angedeutet, durch die Verwendung von Distanzelementen 150 eingestellt werden, welche die Spaltbreite h bestimmen. Bei sehr kleinen Spaltbreiten kann das Verteilerelement 126 mit seiner Verteileroberfläche 130 auch unmittelbar auf die Testfeldoberfläche 124 aufgebracht werden, so dass der Kapillarspalt 132 lediglich durch Oberflächenrauigkeiten der Testfeldoberfläche 124 und/oder der Verteileroberfläche 130 bedingt ist. Somit kann der erfindungsgemäße Kapillarspalt 132 auch durch unmittelbares Aufsetzen der Verteileroberfläche 130 auf die Testfeldoberfläche 124 hergestellt sein, was noch vom erfindungsgemäßen Begriff des Kapillarspalts 132 umfasst sein soll. Im Gegensatz zu gängigen Teststreifen mit Kapillar-Konzepten, wie beispielsweise für elektrochemische Teststreifen, entsteht auf diese Weise ein nicht-offenes System. Dies ist durch das Ausführungsbeispiel gemäß den Figuren 2A und 2B angedeutet, kann jedoch auch beispielsweise in den Ausführungsbeispielen gemäß den Figuren 1 und/oder 3 realisiert sein. Die freie Testfeldoberfläche 124, also der Bereich der Testfeldoberfläche 124, welcher vom Kapillarspalt 132 aus für die Probe 114 zugänglich ist, kann beispielsweise eine Fläche von 5 mm . 4 mm aufweisen.

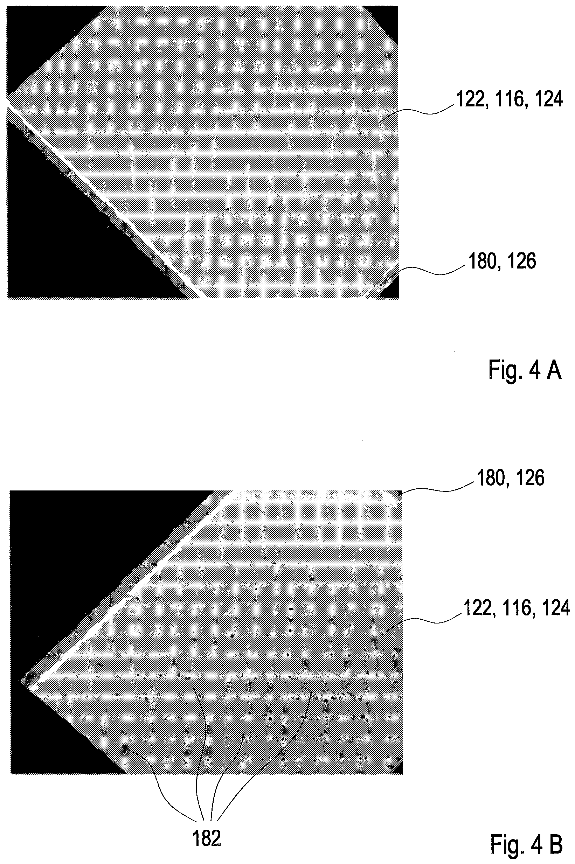

Ein Beispiel für eine effiziente Benetzung durch die Verwendung eines Kapillarspalts 132 ist in den Figuren 4A und 4B dargestellt. Dabei wurde ein Testfeld 116 mit einer Fläche von 5 mm . 4 mm in Kontakt mit einem Objektträger 180 aus Glas gebracht, derart, dass dieser Objektträger 180 die Testfeldoberfläche 124 des Testfelds 116 vollständig bedeckt. Der Objektträger 180 übernimmt in diesem einfachen Versuch die Rolle des Verteilerelements 126. Die Aufnahmen in den Figuren 4A und 4B zeigen einen Schichtaufbau, welcher in die Darstellungsebene hinein aufgezählt die folgenden Schichten umfasst: eine POKALON®-Folie als Trägerelement 122, ein Testfeld 116 und den Objektträger 180. Die Bilder sind dabei aus einem Winkel von 45° beleuchtet. Figur 4A zeigt das derartige System vor dem Einbringen von Wasser, wo hingegen Figur 4B den Aufbau nach der Benetzung mit Wasser zeigt. Für diese Benetzung wurde am Rand des Aufbaus ca. 1 µl Wasser als Tropfen aufgebracht. Dieser Tropfen verteilte sich innerhalb von ca. 1 s über die Anordnung. Die Benetzungszeit spielt also, wie unten weiter ausgeführt wird, eine im Vergleich zur sonstigen Kinetik des Testelements, eine untergeordnete Rolle, ist also in der Regel nicht der geschwindigkeitsbestimmende Schritt, beispielsweise im Vergleich zu den unten näher beschriebenen Diffusionsprozessen. Die Aufnahme in Figur 4B entstand ca. 5 s nach Aufbringen des Wassertropfens. Deutlich sind hier über die gesamte Fläche verteilte Poren 182 zu erkennen, welche die Benetzung mit Wasser belegen. Im Gegensatz dazu benetzte ein Teststreifen mit einem POKALON®-Trägerelement 122 und einem darauf aufgebrachten Testfeld 116, ohne dass ein darauf aufgebrachter Objektträger 180 verwendet wurde, innerhalb der genannten 5 s bisweilen nur schlecht, sofern nicht zusätzliche Maßnahmen ergriffen werden, wie beispielsweise die Verwendung von Spreitnetzen.

Das Experiment in den Figuren 4A und 4B zeigt somit ein Ausführungsbeispiel eines Testelements 110, bei welchem die Verteileroberfläche 130 unmittelbar auf die Testfeldoberfläche 124 aufgelegt ist, also beispielsweise analog zu dem Ausführungsbeispiel gemäß den Figuren 2A und 2B. Wie oben beschrieben, lassen sich jedoch auch die anderen Ausführungsbeispiele in den Figuren 1 und 3 entsprechend modifizieren. Ein derartiges Testelement 110, wie beispielsweise in den Figuren 3A und 3B, stellt somit ein "geschlossenes" System dar, bei welchem der Kapillarspalt 132 lediglich durch Oberflächenrauigkeiten erzeugt ist. Wie oben dargestellt, lassen sich jedoch optional zusätzlich Distanzelemente 150 einsetzen. Die Messung kann, wie oben beschrieben, aus unterschiedlichen Richtungen erfolgen, beispielsweise parallel zur lateralen Erstreckung der Testfeldoberfläche 124 und/oder senkrecht hierzu, wie beispielsweise oben dargestellt wurde. Auch Kombinationen der Messmethoden sind möglich. Durch den vorzugsweise gleichmäßigen Abstand zwischen Verteileroberfläche 130 und Testfeldoberfläche 124 wird an den benetzten Stellen das Flüssigkeitsvolumen pro Flächeneinheit kaum variieren, und eine robuste Messung bei einem entsprechend homogenen Testfeld 116 wird ermöglicht. Auch die optionale Geschlossenheit des Systems trägt zur Robustheit bei. Ferner garantieren die vorzugsweise parallelen Oberflächen 124, 130 in vielen Fällen eine vollständige Absorption des Probenmaterials, so dass auch kleine Probenvolumina sehr effektiv ausgenutzt werden können.

Insgesamt können die vorgeschlagenen Testelemente 110 mit Spaltbreiten h von 10 $\mu$m oder weniger, insbesondere in Form von geschlossenen Systemen mit unmittelbar auf die Testfeldoberfläche 124 aufgebrachter Verteileroberfläche 130, zu einer erheblichen Verbesserung des Spreitverhaltens führen. Zudem können derartige Testelemente 110 Platz sparend ausgestaltet sein. Durch die schnelle Benetzung der Probe 114 gleichmäßig über die benetzte Fläche, kann eine schnelle Kinetik der Analyt-spezifischen Reaktion erfolgen. Dies kann insbesondere für Fluoreszenzmessungen einen Vorteil bedeuten, bei denen in offenen Systemen, wie unten näher ausgeführt wird, die Nachweisstoffe, insbesondere Fluorphore, durch Wegdiffusion nicht oder nur noch ungenügend registriert werden können, was zu Änderungen im Signalverhalten führen kann.

Wie oben ausgeführt, macht sich der Effekt des Kapillarspalts 132 besonders positiv bemerkbar, wenn das Testfeldmaterial 118 des Testfelds 116 eine geringe Korngröße aufweist, vorzugsweise eine Korngröße im Bereich zwischen 50 nm und 5 $\mu$m. Eine derartig geringe mittlere Korngröße kann beispielsweise durch Mahlen erzielt werden.

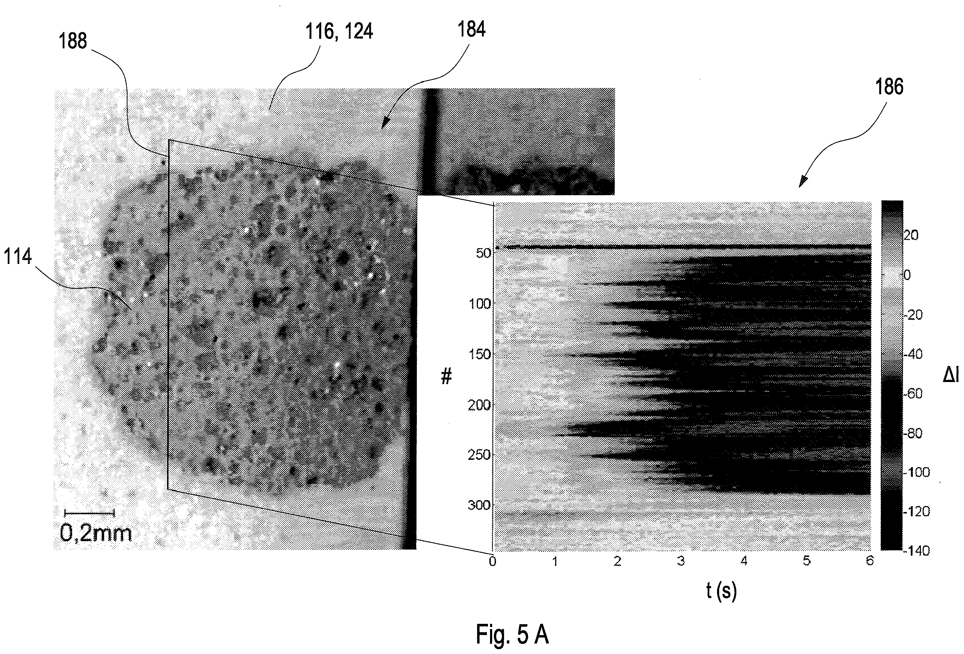

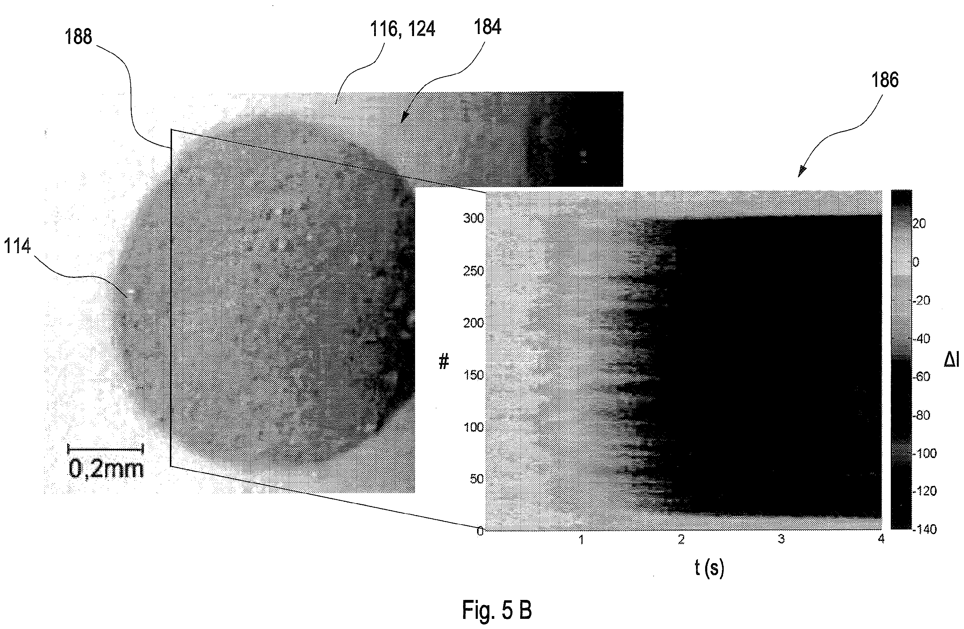

Diese Korngröße in dem bevorzugten Bereich hat unterschiedliche Effekte zur Folge. Ein erster Effekt ist in Vergleichsversuchen in den Figuren 5A und 5B dargestellt. Diese Vergleichsversuche zeigen Testfelder 116 mit einer Testfeldoberfläche 124, auf welche ein Tropfen einer Probe 114 aufgebracht ist. Dabei zeigen die Teilbilder 184 in den Figuren 5A und 5B jeweils Mikroskopaufnahmen der Testfeldoberfläche 124, wohingegen die Teilbilder 186 eine Grauwertänderung in den Mikroskopaufnahmen 184 entlang einer Schnittlinie 188 durch den Tropfen der Probe 114 zeigen. Als Probe wurde dabei eine Testflüssigkeit mit einer Konzentration von 500 mg/dl Glucose verwendet.

In den Teilbildern 186 ist dabei auf der vertikalen Achse in willkürlichen Einheiten die mit # bezeichnete Pixelposition entlang der Schnittlinie 188 aufgetragen. Die horizontale Achse gibt die Zeit t in Sekunden nach Aufbringen der Probe 114 an. In dem Teilbild 186 sind dabei jeweils die Grauwertänderungen dargestellt. Rechts in diesem Teilbild ist eine Skala dargestellt, welche die Grauwertänderung $\Delta$I in willkürlichen Einheiten angibt. Dabei zeigt Figur 5A eine Testfeldoberfläche 124 mit einem ungemahlenen Testfeldmaterial 118, wie es derzeit in handelsüblichen Teststreifen verwendet wird. Figur 5B zeigt hingegen ein Testfeld 116 mit einem gemahlenen Testfeldmaterial, dessen Korngrößen in dem oben angegebenen bevorzugten Bereich liegen.

Ohne auf numerische Details der Messung einzugehen, zeigen insbesondere die Teilbilder 186 in den Figuren 5A und 5B im unmittelbaren Vergleich, dass das Mahlen des Testfeldmaterials 118 zu einer deutlich homogeneren zeitlichen Änderung der Remissionscharakteristik entlang der Schnittlinie 188 führt. Dementsprechend erfolgt das Einsetzen der Reaktion, welche für den Nachweis des nachzuweisenden Analyten spezifisch ist, in dem Ausführungsbeispiel gemäß Figur 5B entlang der Schnittlinie 188 nahezu zeitgleich, wohingegen in dem Versuch mit ungemahlenem Testfeldmaterial 118 gemäß Figur 5A lokal ein starker zeitlicher Versatz des Reaktionseinsatzes zu verzeichnen ist. So kann ein zeitlicher Versatz zwischen einzelnen Orten entlang der Schnittlinie 188 eintreten, welcher bis zu 3 s oder mehr betragen kann. Auch sind Orte entlang der Schnittlinie 188 zu beobachten, an welchen die Reaktion instantan eintritt, sowie Orte, an welchen die Reaktion überhaupt nicht abzulaufen scheint.

Insgesamt lässt sich somit feststellen, dass als erster positiver Effekt der Verwendung eines gemahlenen Testfeldmaterials 118 eine Vergleichmäßigung des Ablaufs der Analyt-spezifischen Reaktion zu beobachten ist, sowie insgesamt eine Homogenisierung dieser Reaktion über die benetzte Testfeldoberfläche 124 hinweg.

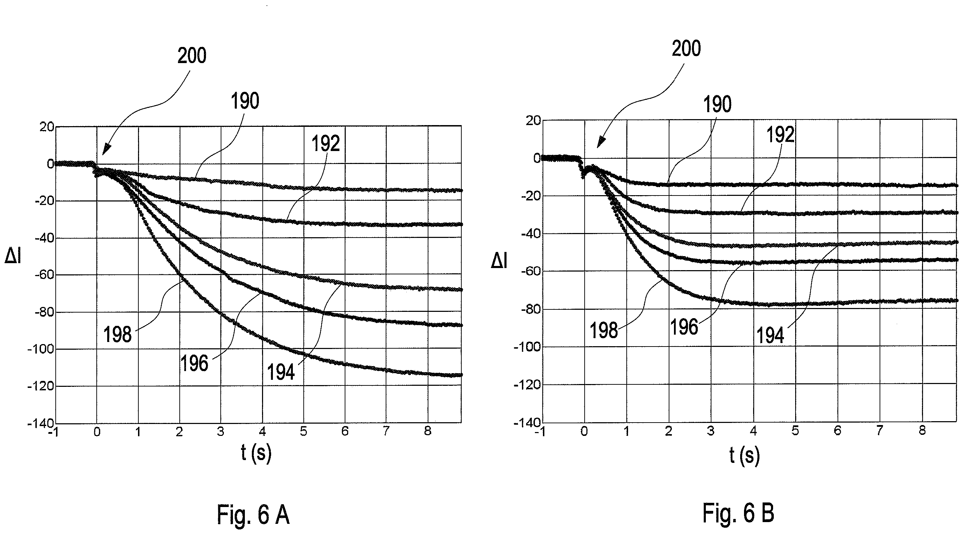

Weiterhin wurden die in den Figuren 5A und 5B gezeigten Versuche auch mit Testelementen 110 durchgeführt, welche einen erfindungsgemäßen Kapillarspalt 132 aufwiesen. Einige dieser Experimente sind exemplarisch in den Figuren 6A und 6B gezeigt. Dabei zeigen diese Figuren jeweils die Grauwertänderung $\Delta I$ bei einer Remissionsmessung in willkürlichen Einheiten als Funktion der Zeit t in Sekunden. Die Messwerte wurden dabei über die Fläche gemittelt. Figur 6A zeigt dabei Experimente mit Testelementen 110 mit nichtgemahlenen Testfeldmaterialien 118, und Figur 6B zeigt Experimente mit gemahlenen Testfeldmaterialien 118 mit Korngrößen im oben beschriebenen bevorzugten Bereich zwischen 50 nm und 5 $\mu$m. Beide Experimente wurden an Testelementen 110 mit Spaltbreiten h von ca. 10 $\mu$m durchgeführt. Dabei wurden unterschiedliche Glucose-Kontrolllösungen verwendet, mit Konzentrationen von 0 mg/dl (Kurven 190), 90 mg/dl (Kurven 192), 200 mg/dl (Kurven 194), 300 mg/dl (Kurven 196) und 500 mg/dl (Kurven 198).

Zunächst ist in beiden Figuren 6A und 6B erkennbar, dass ab dem Zeitpunkt des Aufbringens der Probe 114 (in Figur 6A und 6B mit der Bezugsziffer 200 bezeichnet), welcher willkürlich als Zeitpunkt "Null" definiert wurde, eine Signaländerung eintritt, welche sich schließlich einem für die Konzentration charakteristischen Endwert nähert. Wie aus einem Vergleich der Figur 6B, in welcher Experimente mit gemahlenem Testfeldmaterial 118 dargestellt sind, mit den Kurven mit ungemahlenem Testfeldmaterial 118 in Figur 6A hervorgeht, stellt sich bei Testelementen 110 mit gemahlenem Testfeldmaterial 118 der Endwert überraschenderweise erheblich schneller ein als bei Experimenten mit ungemahlenem Testfeldmaterial 118. Ähnliche Vergleichsversuche wurden auch mit unterschiedlichen Spaltbreiten h durchgeführt. In Tabelle 1 sind die Ergebnisse dieser Vergleichsversuche nochmals grob zusammengefasst.

*Tabelle 1: Endpunkte der Grauwertänderung für unterschiedliche Spaltbreiten und gemahlene und nicht-gemahlene Testfeldmaterialien*

| Konzentrationen [mg/dl] | 10 $\mu$m Spaltbreite, Testfeldmaterial nicht gemahlen | 10 $\mu$m Spaltbreite, Testfeldmaterial gemahlen | 100 $\mu$m Spaltbreite, Testfeldmaterial gemahlen |
|---|---|---|---|
| 90 | 5,8 | 3 | 3,8 |
| 200 | 6,6 | 3,4 | 5,4 |
| 300 | 6,6 | 3 | 5,4 |
| 500 | 6,2 | 3,8 | 5,8 |

Die erste Spalte der Tabelle 1 zeigt dabei jeweils die Konzentrationen der Kontrolllösungen. Die zweite Spalte zeigt die Endpunkte in Sekunden, also die Zeitpunkte, zu denen sich die Endwerte eingestellt haben, für Testelemente 110 mit Spaltbreiten von ca. 10 $\mu$m und ungemahlenem Testfeldmaterial 118, entsprechend der Kurven in Figur 6A. Die zweite Spalte zeigt Experimente mit Spaltbreiten von ca. 10 $\mu$m bei gemahlenem Testfeldmaterial 118, entsprechend den Kurven in Figur 6B. Die dritte Spalte zeigt Endpunkte für Testelemente 110 mit Spaltbreiten von ca. 100 $\mu$m, wobei ebenfalls gemahlene Testfeldmaterialien 118 verwendet wurden.

Die Ergebnisse in Tabelle 1 zeigen deutlich, dass sich gemahlene Testfeldmaterialien 118 und Spaltbreiten von 10 $\mu$m oder weniger als optimal erweisen. Für derartige Testelemente 110 liegen die Endpunkte nahe bei 3 s, wohingegen für andere Versuchsaufbauten erheblich höhere Endpunkte verzeichnet werden. Somit ist insgesamt festzustellen, dass die oben dargestellten bevorzugten Spaltbreiten, insbesondere in Kombination mit gemahlenen Testfeldmaterialien 118 mit den dargestellten bevorzugten Korngrößen, zu einer erheblichen Beschleunigung der Messung führen können.

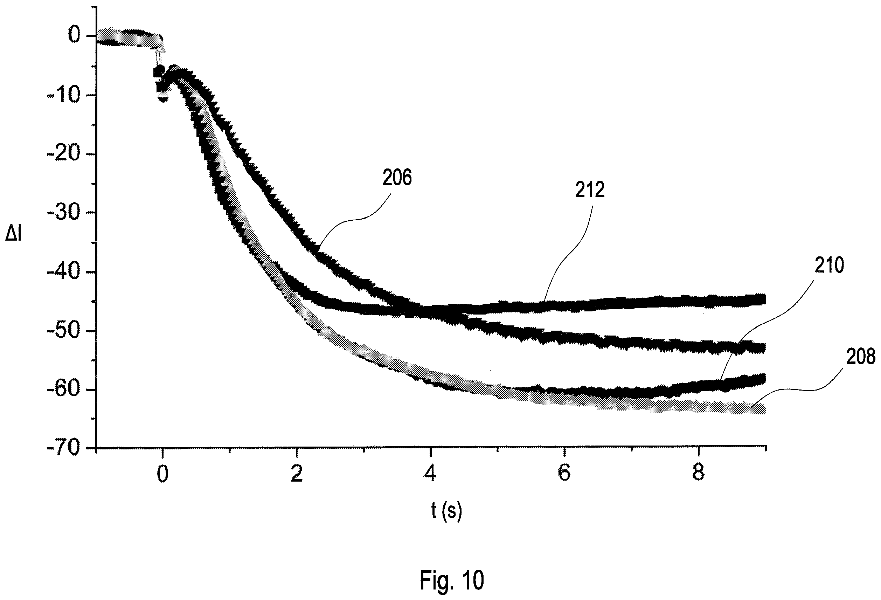

In Figur 10 ist, analog zu den Messungen in Figur 6A und Figur 6B, eine Versuchsreihe dargestellt, in welcher verschiedene Spaltbreiten miteinander verglichen wurden. Aufgetragen ist wiederum die Grauwertänderung $\Delta I$ in willkürlichen Einheiten, als Funktion der Zeit t in s. Dabei wurden jeweils wässrige Glucoselösungen mit einer Konzentration von 200 mg/dl verwendet. Als Vergleichsversuch wurde zunächst ein Testelement 110 ohne Kapillarspalt 132 eingesetzt, also mit einer freien, unbedeckten Testfeldoberfläche 124. Diese Kurve ist in Figur 10 mit der Bezeichnung 206 bezeichnet. Diese Messung beschreibt also herkömmliche Testelemente 110, in welcher ein freier Tropfen auf eine Testfeldoberfläche 124 aufgebracht wird.

Die weiteren Messkurven bezeichnen Experimente mit Testelementen 110 mit Kapillarspalten 132. Dabei bezeichnet Kurve 208 ein Testelement 110 mit einer Spaltbreite von 100 $\mu$m, Kurve 210 ein Testelement 110 mit einer Spaltbreite von 60 $\mu$m und Kurve 212 ein Testelement 110 mit einer Spaltbreite von 10 $\mu$m. Dabei wurden durchgehend Testelemente 110 mit gemahlenen Testfeldmaterialien 118 verwendet. Die Kurve 212 entspricht also der Kurve 194 gemäß Figur 6B.

Abgesehen von weiteren Besonderheiten des quantitativen Verlaufs der Kurven 206 bis 212 zeigen diese Vergleichsergebnisse wiederum den bereits oben beschriebenen Effekt, nämlich, dass bei kleinen Spaltbreiten von 10 $\mu$m oder weniger der Endpunkt der Messungen erheblich schneller erreicht wird als bei größeren Spaltbreiten. So stellt sich der Endpunkt bei der Kurve 212 wiederum nach bereits ca. 2 bis 3 s ein, wohingegen bei den übrigen Kurven 206 bis 210 der Endpunkt erst wesentlich später, nach 5 s oder mehr, erreicht wird. Dies zeigt im direkten Vergleich nochmals deutlich den Vorteil der erfindungsgemäßen kleinen Spaltbreiten bezüglich der Geschwindigkeit der Messungen.

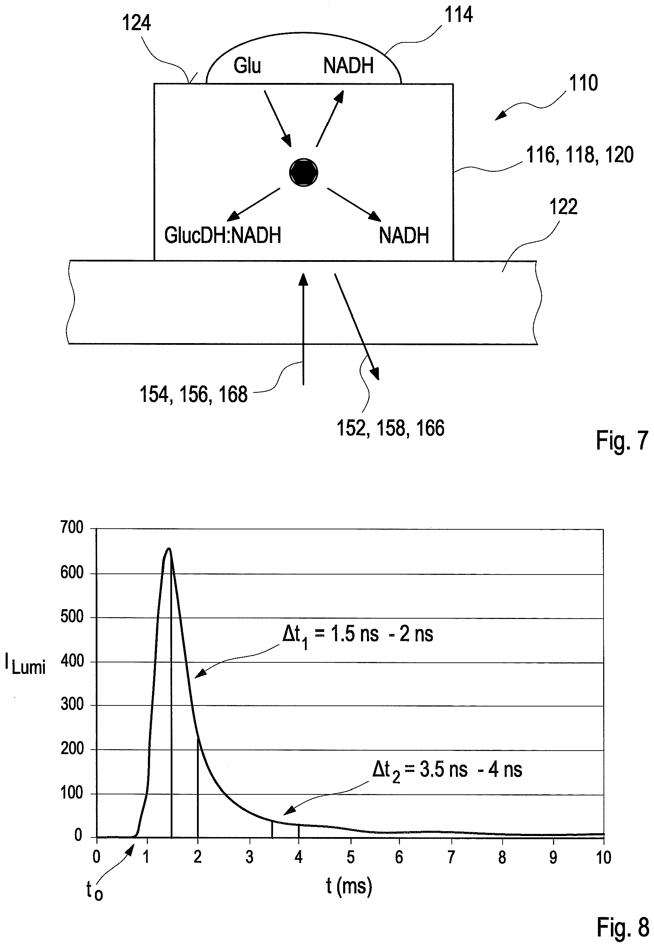

Das Nachweisreagenz 120 kann beispielsweise, wie oben dargestellt, Glucosedehydrogenase (GlucDH) umfassen, welche mit eindringender Glucose (in Figur 7 mit Glu bezeichnet) reagiert und mittels einem Farbstoff eine Farbänderung verursacht. Diese Farbänderung kann durch eine Remissionsmessung, in Figur 7 symbolisiert durch den Beleuchtungsstrahl 156 und den Remissionsstrahl 158, erfasst werden. Alternativ oder zusätzlich kann das Nachweisreagenz 120 jedoch auch Glucosedehydrogenase und NAD+ umfassen, welche unter Einwirkdung von Glucose NADH als Fluorophor bildet. Dieser Fluorophor kann photometrisch nachgewiesen werden, was in Figur 7 durch die Strahlen 154, 156 und 152, 158 dargestellt ist. Gleichzeitig wurden in den Versuchen jedoch auch, was bislang bei Testgeräten nicht erfolgte, Lumineszenzmessungen durchgeführt, was in Figur 7 durch die Strahlen 154, 168 und 152, 166 dargestellt ist. In beiden Fällen kann NADH als Fluorophor wirken.

NADH kann jedoch, wie in Figur 7 symbolisch dargestellt, in unterschiedlicher Form vorliegen. Zum einen kann NADH im Inneren des Testfelds 116 in komplexierter Form vorliegen, beispielsweise als Komplex mit Glucosedehydrogenase (GlucDH), der in Figur 7 mit GlucDH:NADH bezeichnet ist. In geringerem Umfang kann NADH in dem Testfeld 116 auch in freier Form vorliegen. Gleichzeitig kann NADH jedoch auch in die Probe 114 hineindiffundieren und dort in freier Form vorliegen, was in Figur 7 ebenfalls symbolisch dargestellt ist.

Um zwischen gebundenem NADH (GlucDH:NADH) und freiem NADH zu unterscheiden, eignen sich insbesondere zeitaufgelöste Fluoreszenzmessungen. Derartige zeitaufgelöste Fluoreszenzmessungen können die Tatsache nutzen, dass die Fluoreszenz von freiem NADH eine Lebensdauer $\tau_1$ von ca. 0,4 ns aufweist, wohingegen komplexiertes NADH (GlucDH:NADH) eine Lebensdauer $\tau_2$ von ca. 3 ns aufweist. Die Lumineszenz weist also insgesamt ein Abklingverhalten auf, welches sich aus beiden Lumineszenzen additiv zusammensetzt:

$$I_{Lumi} = A_1 \cdot \exp[- t / \tau_1] + A_2 \cdot \exp[- t / \tau_2].$$

Dabei bezeichnet $I_{Lumi}$ die Lumineszenzintensität, t bezeichnet die Zeit, $\tau_1$ und $\tau_2$ bezeichnen die Abklingzeiten der jeweiligen Lumineszenzen, und $A_1$ und $A_2$ bezeichnen Anfangsintensitäten. Das Abklingverhalten einer derartigen kombinierten Lumineszenz ist in Figur 8 dargestellt. Dabei ist die Lumineszenzintensität $I_{Lumi}$ in willkürlichen Einheiten als Funktion der Zeit t in ns aufgetragen. Zum Zeitpunkt $t_0$ findet eine Anregung statt.

In Figur 8 ist zudem eine Messung in zwei Zeitfenstern dargestellt. Ein erstes Zeitfenster $\Delta t_1$ liegt dabei willkürlich in einem Intervall zwischen 1,5 und 2 ns. Dieses Zeitfenster wurde derart gewählt, dass zu Beginn dieses Zeitfensters die Anregung weitgehend abgeschlossen ist, wobei sich die Lumineszenz innerhalb dieses Zeitfensters $\Delta t_1$ noch weitgehend aus beiden Lumineszenzen zusammensetzt, da der Endpunkt des Intervalls noch nicht wesentlich größer ist als die kleinere der beiden Lebensdauern, in diesem Fall $\tau_1$.

Ein zweites Zeitfenster $\Delta t_2$ wurde zwischen 3,5 ns und 4 s gewählt. Da der Anfangspunkt dieses Zeitfensters von 3,5 s vergleichsweise groß ist gegenüber der Fluoreszenzlebensdauer $\tau_1$ des freien NADHs von 0,4 s, setzt sich die Lumineszenz in diesem zweiten Zeitfenster $\Delta t_2$ praktisch ausschließlich aus der Lumineszenz des gebundenen NADHs (GlucDH:NADH) zusammen, welche eine Lebensdauer von ca. 3 ns aufweist.

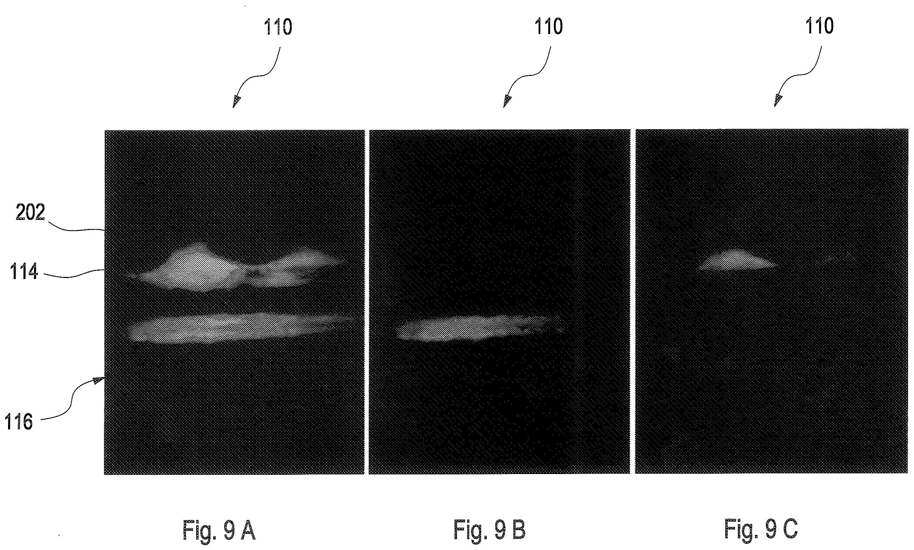

Mittels derartiger zeitaufgelöster Messungen lässt sich somit die Lumineszenz des freien NADHs von der Lumineszenz des gebundenen NADH trennen. Zum Nachweis, dass dieses Prinzip der Trennung funktioniert, wurden an herkömmlichen Testelementen 110 zeitaufgelöste und ortsaufgelöste Lumineszenzmessungen durchgeführt, welche in den Figuren 9A bis 9C dargestellt sind. Dabei zeigt Figur 9A eine Messung im Zeitfenster $\Delta t_1$ zwischen 1,5 und 2 ns, Figur 9B zeigt eine Messung im Zeitfenster $\Delta t_2$ zwischen 3,5 und 4 ns, und Figur 9C zeigt ein Differenzbild aus den Figuren 9A und 9B.

Für diese Versuche, welche lediglich das Messprinzip erläutern sollen, wurden kommerzielle Testelemente 110 verwendet. Die Testelemente 110 umfassen über den Testfeldern 116 Spreitnetze 202, welche insbesondere bei der Darstellung gemäß Figur 9A deutlich zu erkennen sind. Zwischen den Testfeldern 116 und den Spreitnetzen 202 bildete sich somit eine flüssige Phase der Probe 114 aus.

Wie aus der Darstellung gemäß Figur 9A hervorgeht, findet im Zeitfenster $\Delta t_1$ Lumineszenz sowohl in der festen Phase des Testfelds 116 als auch in der flüssigen Phase der darüber liegenden Probe 114 statt. Im Zeitfenster $\Delta t_2$, dargestellt in Figur 9B, ist hingegen lediglich noch Lumineszenz aus dem Bereich des Testfelds 116 zu verzeichnen, nicht mehr hingegen aus dem Bereich der flüssigen Probe 114. Durch die in Figur 9C dargestellte Differenzbildung lässt sich somit aus diesen Messungen die Lumineszenz des freien NADH mit der kürzeren Fluoreszenzlebensdauer $\tau_1$ herausrechnen. Figur 9C zeigt also Fluoreszenz, welche nahezu ausschließlich durch freies NADH bedingt ist.

Die theoretischen Überlegungen und diese Messungen, welche jedoch unabhängig von der Erfindung sind und an deren Korrektheit und Vollständigkeit die Erfindung nicht gebunden ist, zeigen, dass Diffusionseffekte des freien Fluorophors NADH, beziehungsweise bei Verwendung anderer Arten von Nachweisstoffen dieser Nachweisstoffe, beim Nachweis eine erhebliche Rolle spielen können. Da eine Diffusion in die flüssige Phase der Probe 114 in erheblichem Maße stattfindet, können die zeitlichen Verläufe dieser Diffusion die Messungen erheblich beeinträchtigen. Hierdurch lassen sich sowohl die Messungen in den Figuren 6A und 6B und in Figur 10 als auch die Ergebnisse in Tabelle 1 zumindest qualitativ erläutern. Zu Beginn der Benetzung mit der Probe 114 laufen mehrere Prozesse ab, zwischen denen sich erst ein Gleichgewicht einstellen muss, bevor ein stabiler Endpunkt in den Messungen, beispielsweise gemäß den Figuren 6A und 6B erreicht ist. Einer dieser Prozesse stellt die Diffusion des Nachweisstoffs NADH in die flüssige Probe dar. Diese Diffusion stellt sich aufgrund eines Konzentrationsunterschiedes zwischen dem Testfeld 116 und der flüssigen Probe 114 ein. Erst wenn sich ein Diffusionsgleichgewicht eingestellt hat, liegen stabile Konzentrationsverhältnisse vor, welche zu einem stabilen Endpunkt in den Figuren 6A und 6B gezeigten Messungen führen. Durch die geringere Schichtdicke von 10 $\mu$m im Vergleich zu den in Tabelle 1 gezeigten 100 $\mu$m ist die Menge der über der Testfeldoberfläche 124 zur Verfügung stehenden Flüssigkeitssäule der Probe 114 bei den kleinen Spaltbreiten geringer, so dass eine geringere Zeitdauer bis zum Einstellen des Fluoreszenzgleichgewichts verstreicht. Insbesondere bei den in Figur 7 angedeuteten rückseitigen Remissionsmessungen, welche auch in den Figuren 6A und 6B aufgetragen sind, wird in der Regel lediglich NADH in freier oder gebundener Form innerhalb des Testfelds 116 gemessen, nicht hingegen freies NADH innerhalb der flüssigen Probe 114. Eine schnellere Einstellung des Diffusionsgleichgewichts bei geringeren Spaltbreiten führt somit auch dazu, dass sich der Endpunkt der photometrischen Remissionsmessungen schneller einstellt als bei höheren Spaltbreiten.

Optional lässt sich dieser Effekt bei einer erfindungsgemäßen Vorrichtung 112 und in einem erfindungsgemäßen Verfahren nutzen, um die Zuverlässigkeit der Messungen zu erhöhen. Dies kann dadurch erfolgen, dass bei der Auswertung der Messungen der Anteil des Nachweisstoffs in der Probe 114 außerhalb des Testfelds 116 berücksichtigt wird, beispielsweise der Anteil freien NADHs in der flüssigen Blutprobe 114. Wie oben dargestellt, kann dies beispielsweise durch eine zeitaufgelöste Messung erfolgen, beispielsweise eine zeitaufgelöste Messung in unterschiedlichen Zeitfenstern, welche die unterschiedlichen Abklingverhalten der Nachweisstoffe innerhalb des Testfelds 116 und innerhalb der flüssigen Probe 114 berücksichtigen. Da die meisten Nachweisreaktionen theoretisch und empirisch gut untersucht sind, sind in der Regel auch die Abklingverhalten der Nachweisstoffe in unterschiedlichen Umgebungen bekannt.

Ist jedoch der Nachweisstoff in der Probe 114 außerhalb des Testfelds 116 zumindest qualitativ, vorzugsweise jedoch quantitativ, bestimmt, beispielsweise in Relation zum Nachweisstoff innerhalb des Testfelds 116, so lässt sich die Messgenauigkeit des Nachweises des Analyten hierdurch verbessern. Zum einen lässt sich der Messzeitpunkt anpassen, da wie oben dargestellt, die Einstellung des Endpunkts von der Spaltbreite abhängt, welche jedoch wiederum mit der absoluten Länge an Nachweisstoff in der flüssigen Probe 114 über der Testfeldoberfläche 124 korreliert. Auf diese Weise lässt sich beispielsweise der Messzeitpunkt, welcher nach dem Endpunkt liegen sollte, entsprechend wählen, beispielsweise automatisch. Alternativ oder zusätzlich kann jedoch auch eine Korrektur der Messergebnisse stattfinden, da der Nachweisstoff innerhalb der flüssigen Probe 114 außerhalb des Testfelds 116, wie oben dargestellt, in der Regel für den Nachweis des Analyten nicht mehr zur Verfügung steht. Ist dieser Anteil bestimmt, so kann beispielsweise mittels entsprechender Korrekturfaktoren eine Korrektur der Testergebnisse vorgenommen werden.

Zur Umsetzung eines Messverfahrens, welches den Nachweisstoff in der Probe 114 außerhalb des Testfelds 116 berücksichtigt, beispielsweise gemäß einer oder beiden der oben beschriebenen Optionen, kann die Vorrichtung 112, beispielsweise gemäß Figur 1, insbesondere eine Kalibrationsvorrichtung 204 umfassen. Diese Kalibrationsvorrichtung 204, welche in Figur 1 lediglich symbolisch angedeutet ist und dort optional und bidirektional mit der photometrischen Messvorrichtung 160 und der Lumineszenz-Messvorrichtung 170 verbunden ist, kann beispielsweise auch ganz oder teilweise in eine zentrale Steuerung der Vorrichtung 112 integriert sein. Beispielsweise kann die Kalibrationsvorrichtung 204 ganz oder teilweise in einem Datenverarbeitungsgerät integriert sein und kann ganz oder teilweise programmtechnisch umgesetzt sein. Die Kalibrationsvorrichtung 204 kann beispielsweise einen oder mehrere Datenspeicher umfassen, in welchen Korrekturfaktoren oder andere Korrekturalgorithmen hinterlegt sind, so dass eine Auswertung der Messung unter Berücksichtigung des Nachweisstoffs in der Probe 114 außerhalb des Testfelds 116 erfolgen kann. Auf diese Weise lassen sich durch das erfindungsgemäße Verfahren und die erfindungsgemäße Ausgestaltung der Vorrichtung 112 beispielsweise Kalibrationen auf eine Spaltbreite h durchführen, so dass produktionstechnische Schwankungen dieser Spaltbreite h durch Online-Kalibrationen ausgeglichen werden können. Die Kalibration kann dabei beispielsweise vor der eigentlichen Messung und/oder während der eigentlichen Messung und/oder nach der eigentlichen Messung durchgeführt werden.

Bezugszeichenliste

110    Testelement zum Nachweis eines Analyten in einer Probe
112    Vorrichtung zum Nachweis eines Analyten in einer Probe
114    Blutstropfen, Probe
116    Testfeld

| 118 | Testfeldmaterial |
|---|---|
| 120 | Nachweisreagenz |
| 122 | Trägerelement |
| 124 | Testfeldoberfläche |
| 126 | Verteilerelement |
| 128 | Folienelement |
| 130 | Verteileroberfläche |
| 132 | Kapillarspalt |
| 134 | Nachweisteil |
| 136 | Koppelteil |
| 138 | Aufnahme |
| 140 | oberes Haltestück |
| 142 | unteres Haltestück |
| 144 | Koppelkörper |
| 146 | Lichtleiter |
| 148 | Lichtleiter |
| 150 | Distanzelemente |
| 152 | Nachweislicht |
| 154 | Abfragelicht |
| 156 | Beleuchtungsstrahl |
| 158 | Remissionsstrahl |
| 160 | photometrische Messvorrichtung |
| 162 | Lichtquelle |
| 164 | Detektor |
| 166 | Lumineszenzlicht |
| 168 | Anregungsstrahl |
| 170 | Lumineszenz-Messvorrichtung |
| 172 | Lichtquelle |
| 174 | Detektor |
| 176 | Nachweisbereich |
| 178 | Applikationsstelle |
| 180 | Objektträger |
| 182 | Poren |
| 184 | Mikroskopaufnahme |
| 186 | Grauwertänderung |
| 188 | Schnittlinie |
| 190 | 0 mg/dl |
| 192 | 90 mg/dl |
| 194 | 200 mg/dl |
| 196 | 300 mg/dl |
| 198 | 500 mg/dl |
| 200 | Aufbringen Probe |
| 202 | Spreitnetze |
| 204 | Kalibrationsvorrichtung |
| 206 | ohne Kapillarspalt |
| 208 | Spaltbreite 100 $\mu$m |
| 210 | Spaltbreite 60 $\mu$m |
| 212 | Spaltbreite 10 $\mu$m |

**Patentansprüche**

1. Vorrichtung (112) zum Nachweis mindestens eines Analyten in einer flüssigen Probe (114), insbesondere zum Nachweis mindestens eines Metaboliten in einer Körperflüssigkeit, wobei die Vorrichtung (112) mindestens ein Testelement (110) umfasst, wobei das Testelement (110) mindestens ein Testfeld (116) mit einer Testfeldoberfläche (124) zum Aufbringen der Probe (114) umfasst, wobei das Testfeld (116) mindestens ein Nachweisreagenz (120) aufweist, welches eingerichtet ist, um bei Anwesenheit des Analyten eine nachweisbare Reaktion durchzuführen, wobei das Nachweisreagenz (120) derart eingerichtet ist, dass bei der nachweisbaren Reaktion im Inneren des Testfeldes (116) mindestens ein Nachweisstoff gebildet wird, wobei die Vorrichtung eingerichtet ist, um den Nachweisstoff in der Probe (114) im Inneren des Testfelds (116) zu bestimmen, wobei der Nachweisstoff durch Diffusion auch außerhalb des Testfeldes (116) innerhalb der Probe (114) vorliegt, wobei die Vorrichtung (112) weiterhin mindestens eine Kalibrationsvorrichtung (204) umfasst, wobei die Kalibrationsvorrichtung (204) eingerichtet ist, um den Nachweisstoff in der Probe (114) außerhalb des Testfelds (116) zu bestimmen, wobei die Vorrichtung (112) weiterhin eingerichtet ist, um den Nachweis des Analyten unter Berücksichtigung der Bestimmung des Nachweisstoffes in der Probe (114) innerhalb des Testfelds (116) und der Bestimmung des Nachweisstoffs in der Probe (114) außerhalb des Testfelds (116) durchzuführen, wobei die Kalibrationsvorrichtung (204) zu diesem Zweck ein Datenverarbeitungsgerät umfasst, wobei hinterlegte Korrekturfaktoren angewandt werden, welche die Diffusion des Nachweisstoffs in die Probe (114) außerhalb des Testfelds (116) berücksichtigen.

2.  Vorrichtung (112) nach dem vorhergehendenAnspruch, wobei die Bestimmung des Nachweisstoffs in der Probe (114) außerhalb des Testfelds (116) direkt erfolgt, wobei unmittelbar die Anwesenheit des Nachweisstoffs qualitativ und/oder quantitativ detektiert wird.

3.  Vorrichtung (112) nach einem der vorhergehenden Ansprüche, wobei die Bestimmung des mindestens einen Nachweisstoffs in der Probe (114) außerhalb des Testfelds (116) indirekt erfolgt, wobei eine Abnahme des Nachweisstoffs und/oder einer Verbindung des Nachweisstoffs bzw. einer den Nachweisstoff enthaltenden Substanz qualitativ und/oder quantitativ nachgewiesen wird.

4.  Vorrichtung (112) nach einem der vorhergehenden Ansprüche, wobei die Kalibrationsvorrichtung (204) eingerichtet ist, um ein Nachweisverfahren durchzuführen, welches darauf beruht, dass der Nachweisstoff in der Probe (114) außerhalb des Testfelds (116) und der Nachweisstoff in dem Inneren des Testfelds (116) unterschiedliche Eigenschaften hinsichtlich einer Wechselwirkung mit einem Abfragelicht und/oder hinsichtlich der Aussendung eines Nachweislichts aufweisen.

5.  Vorrichtung (112) nach einem der vorhergehenden Ansprüche, wobei die Kalibrationsvorrichtung (204) dazu eingerichtet ist, um den Nachweisstoff in der Probe (114) außerhalb des Testfelds (116) mittels mindestens einer zeitaufgelösten Messung nachzuweisen.

6.  Vorrichtung (112) nach dem vorhergehenden Anspruch, wobei die Kalibrationsvorrichtung (204) dazu eingerichtet ist, um den Nachweisstoff in der Probe (114) außerhalb des Testfelds (116) mittels mindestens einer zeitaufgelösten optischen Messung nachzuweisen.

7.  Vorrichtung (112) nach dem vorhergehenden Anspruch, wobei die Kalibrationsvorrichtung (204) dazu eingerichtet ist, um den Nachweisstoff in der Probe (114) außerhalb des Testfelds (116) mittels einer Fluoreszenz-Messung nachzuweisen.

8.  Vorrichtung (112) nach einem der vorhergehenden Ansprüche, wobei das Testelement (110) mindestens einen mit dem Testfeld (116) in Verbindung stehenden Kapillarspalt (132) zum Aufnehmen und Verteilen der Probe (114) auf dem Testfeld (116) umfasst, wobei die Kalibrationsvorrichtung (204) eingerichtet ist, um mittels des Nachweises des Nachweisstoffs in der Probe (114) außerhalb des Testfelds (116) eine Kalibration einer Spaltbreite des Kapillarspalts (132) vorzunehmen.

9.  Vorrichtung (112) nach dem vorhergehenden Anspruch, wobei der Kapillarspalt (132) derart eingerichtet ist, dass sich innerhalb des Kapillarspalts eine Schicht der Probe (114) mit einer Schichtdicke von nicht mehr als 50 $\mu$m ausbildet, wobei die Kalibrationsvorrichtung (204) dazu eingerichtet ist, um bei der Bestimmung des Nachweisstoffs in der Probe (114) außerhalb des Testfelds (116) Schwankungen in der Schichtdicke der Probe (114) zu berücksichtigen.

10. Verfahren zum Nachweis mindestens eines Analyten in einer flüssigen Probe (114), insbesondere zum Nachweis mindestens eines Metaboliten in einer Körperflüssigkeit, insbesondere unter Verwendung einer Vorrichtung (112) nach einem der vorhergehenden, eine Vorrichtung (112) betreffenden Ansprüche, wobei mindestens ein Testelement (110) verwendet wird, wobei das Testelement (110) mindestens ein Testfeld (116) mit einer Testfeldoberfläche (124) zum Aufbringen der Probe (114) umfasst, wobei das Testfeld (116) mindestens ein Nachweisreagenz (120) aufweist, welches eingerichtet ist, um bei Anwesenheit des Analyten eine nachweisbare Reaktion durchzuführen, wobei das Nachweisreagenz (120) derart eingerichtet ist, dass bei der nachweisbaren Reaktion im Inneren des Testfeldes (116) mindestens ein Nachweisstoff gebildet wird, wobei die Vorrichtung eingerichtet ist, um den Nachweisstoff in der Probe (114) im Inneren des Testfelds (116) zu bestimmen, wobei der Nachweisstoff durch Diffusion auch außerhalb des Testfeldes (116) innerhalb der Probe (114) vorliegt, wobei der Nachweisstoff in der Probe (114) außerhalb des Testfelds (116) bestimmt wird und wobei der Nachweis des Analyten unter Berücksichtigung der Bestimmung des Nachweisstoffes in der Probe (114) innerhalb des Testfelds (116) und der Bestimmung des Nachweisstoffs in der Probe (114) außerhalb des Testfelds (116) durchgeführt wird, wobei eine Kalibrationsvorrichtung (204) mit einem Datenverarbeitungsgerät verwendet wird, wobei hinterlegte Korrekturfaktoren angewandt werden, welche die Diffusion des Nachweisstoffs in die Probe (114) außerhalb des Testfelds (116) berücksichtigen.

11. Verfahren nach dem vorhergehenden Anspruch, wobei das Testelement (110) mindestens einen mit dem Testfeld (116) in Verbindung stehenden Kapillarspalt (132) zum Aufnehmen und Verteilen der Probe (114) auf dem Testfeld (116) umfasst, wobei der Kapillarspalt (132) derart eingerichtet ist, dass sich innerhalb des Kapillarspalts eine Schicht der Probe (114) mit einer Schichtdicke von nicht mehr als 50 μm ausbildet, wobei bei der Bestimmung des Nachweisstoffs in der Probe (114) außerhalb des Testfelds (116) Schwankungen in der Schichtdicke der Probe (114) berücksichtigt werden.

12. Verfahren nach einem der vorhergehenden Verfahrensansprüche, wobei die Bestimmung des Nachweisstoffs in der Probe (114) außerhalb des Testfelds (116) direkt erfolgt, wobei unmittelbar die Anwesenheit des Nachweisstoffs qualitativ und/oder quantitativ detektiert wird.

13. Verfahren nach einem der vorhergehenden Verfahrensansprüche, wobei die Bestimmung des mindestens einen Nachweisstoffs in der Probe (114) außerhalb des Testfelds (116) indirekt erfolgt, wobei eine Abnahme des Nachweisstoffs und/oder einer Verbindung des Nachweisstoffs bzw. einer den Nachweisstoff enthaltenden Substanz qualitativ und/oder quantitativ nachgewiesen wird.

## Claims

1. Device (112) for the detection of at least one analyte in a liquid sample (114), especially for the detection of at least one metabolite in a body fluid, the device (112) comprising at least one test element (110), the test element (110) comprising at least one test field (116) having a test field surface (124) for the application of the sample (114), the test field (116) having at least one detection reagent (120) configured to carry out a detectable reaction in the presence of the analyte, the detection reagent (120) being configured such that at least one detection substance is formed inside the test field (116) in the detectable reaction, the device being configured to determine the detection substance in the sample (114) inside the test field (116), the detection substance also being present outside the test field (116) within the sample (114) as a result of diffusion, the device (112) further comprising at least one calibration device (204), the calibration device (204) being configured to determine the detection substance in the sample (114) outside the test field (116), the device (112) being further configured to carry out the detection of the analyte with consideration of the determination of the detection substance in the sample (114) within the test field (116) and of the determination of the detection substance in the sample (114) outside the test field (116), the calibration device (204) comprising a data processor for this purpose, wherein stored correction factors taking into account the diffusion of the detection substance into the sample (114) outside the test field (116) are used.

2. Device (112) according to the preceding claim, wherein the determination of the detection substance in the sample (114) outside the test field (116) is performed directly, wherein the presence of the detection substance is immediately detected in a qualitative and/or quantitative manner.

3. Device (112) according to any one of the preceding claims, wherein the determination of the at least one detection substance in the sample (114) outside the test field (116) is performed indirectly, wherein a decrease in the detection substance and/or in a compound of the detection substance or a detection substance-containing substance is detected in a qualitative and/or quantitative manner.

4. Device (112) according to any one of the preceding claims, wherein the calibration device (204) is configured to carry out a detection method based on the detection substance in the sample (114) outside the test field (116) and the detection substance inside the test field (116) having different properties with respect to an interaction with a query light and/or with respect to the emission of a detection light.

5. Device (112) according to any one of the preceding claims, wherein the calibration device (204) is configured to detect the detection substance in the sample (114) outside the test field (116) by means of at least one time-resolved measurement.

6.  Device (112) according to the preceding claim, wherein the calibration device (204) is configured to detect the detection substance in the sample (114) outside the test field (116) by means of at least one time-resolved optical measurement.

7.  Device (112) according to the preceding claim, wherein the calibration device (204) is configured to detect the detection substance in the sample (114) outside the test field (116) by means of a fluorescence measurement.

8.  Device (112) according to any one of the preceding claims, wherein the test element (110) comprises at least one capillary gap (132) in communication with the test field (116) for uptake and distribution of the sample (114) on the test field (116), the calibration device (204) being configured to perform a calibration of a gap width of the capillary gap (132) by means of the detection of the detection substance in the sample (114) outside the test field (116).

9.  Device (112) according to the preceding claim, wherein the capillary gap (132) is configured such that a layer of the sample (114) having a layer thickness of not more than 50 μm is formed within the capillary gap, the calibration device (204) being configured to take into account fluctuations in the layer thickness of the sample (114) when determining the detection substance in the sample (114) outside the test field (116).

10. Method for detecting at least one analyte in a liquid sample (114), especially for detecting at least one metabolite in a body fluid, especially with use of a device (112) according to any of the preceding claims relating to a device (112), at least one test element (110) being used, the test element (110) comprising at least one test field (116) having a test field surface (124) for the application of the sample (114), the test field (116) having at least one detection reagent (120) configured to carry out a detectable reaction in the presence of the analyte, the detection reagent (120) being configured such that at least one detection substance is formed inside the test field (116) in the detectable reaction, the device being configured to determine the detection substance in the sample (114) inside the test field (116), the detection substance also being present outside the test field (116) within the sample (114) as a result of diffusion, the detection substance in the sample (114) outside the test field (116) being determined and the detection of the analyte being carried out with consideration of the determination of the detection substance in the sample (114) within the test field (116) and of the determination of the detection substance in the sample (114) outside the test field (116), a calibration device (204) having a data processor being used, wherein stored correction factors taking into account the diffusion of the detection substance into the sample (114) outside the test field (116) are used.

11. Method according to the preceding claim, wherein the test element (110) comprises at least one capillary gap (132) in communication with the test field (116) for uptake and distribution of the sample (114) on the test field (116), the capillary gap (132) being configured such that a layer of the sample (114) having a layer thickness of not more than 50 μm is formed within the capillary gap, wherein fluctuations in the layer thickness of the sample (114) are taken into account when determining the detection substance in the sample (114) outside the test field (116).

12. Method according to any one of the preceding method claims, wherein the determination of the detection substance in the sample (114) outside the test field (116) is performed directly, wherein the presence of the detection substance is immediately detected in a qualitative and/or quantitative manner.

13. Method according to any one of the preceding method claims, wherein the determination of the at least one detection substance in the sample (114) outside the test field (116) is performed indirectly, wherein a decrease in the detection substance and/or in a compound of the detection substance or a detection substance-containing substance is detected in a qualitative and/or quantitative manner.

**Revendications**

1.  Dispositif (112) pour la détection d'au moins un analyte dans un échantillon liquide (114), notamment pour la détection d'au moins un métabolite dans un liquide corporel, le dispositif (112) comprenant au moins un élément de test (110), l'élément de test (110) comprenant au moins un champ de test (116) comprenant une surface de champ de test (124) pour l'application de l'échantillon (114), le champ de test (116) comprenant au moins un réactif de détection (120), qui est conçu pour réaliser une réaction détectable en présence de l'analyte, le réactif de détection (120) étant conçu de telle sorte qu'au moins une matière de détection soit formée lors de la réaction détectable à l'intérieur du champ de test (116), le dispositif étant conçu pour déterminer la matière de détection dans l'échantillon (114) à l'intérieur du champ de test (116), la matière de détection étant également présente à l'extérieur du champ de test (116) dans l'échantillon (114) par diffusion, le dispositif comprenant en outre au moins un dispositif de calibration (204), le dispositif de calibration (204) étant conçu pour déterminer la matière de détection dans l'échantillon (114) à l'extérieur du champ de test (116), le dispositif (112) étant en outre conçu pour réaliser la détection de l'analyte en prenant en compte la détermination de la matière de détection dans l'échantillon (114) dans le champ de test (116) et la détermination de la matière de détection dans l'échantillon (114) à l'extérieur du champ de test (116), le dispositif de calibration (204) comprenant à cette fin un appareil de traitement des données, dans lequel des facteurs de correction déposés sont appliqués, qui prennent en compte la diffusion de la matière de détection dans l'échantillon (114) à l'extérieur du champ de test (116).

2. Dispositif (112) selon la revendication précédente, dans lequel la détermination de la matière de détection dans l'échantillon (114) à l'extérieur du champ de test (116) a lieu directement, la présence de la matière de détection étant détectée directement qualitativement et/ou quantitativement.

3. Dispositif (112) selon l'une quelconque des revendications précédentes, dans lequel la détermination de ladite au moins une matière de détection dans l'échantillon (114) à l'extérieur du champ de test (116) a lieu indirectement, une diminution de la matière de détection et/ou d'un composé de la matière de détection ou d'une substance contenant la matière de détection étant détectée qualitativement et/ou quantitativement.

4. Dispositif (112) selon l'une quelconque des revendications précédentes, dans lequel le dispositif de calibration (204) est conçu pour réaliser un procédé de détection qui repose sur le fait que la matière de détection dans l'échantillon (114) à l'extérieur du champ de test (116) et la matière de détection à l'intérieur du champ de test (116) présentent des propriétés différentes au regard d'une interaction avec une lumière d'interrogation et/ou au regard de l'émission d'une lumière de détection.

5. Dispositif (112) selon l'une quelconque des revendications précédentes, dans lequel le dispositif de calibration (204) est conçu pour détecter la matière de détection dans l'échantillon (114) à l'extérieur du champ de test (116) au moyen d'au moins une mesure à résolution temporelle.

6. Dispositif (112) selon la revendication précédente, dans lequel le dispositif de calibration (204) est conçu pour détecter la matière de détection dans l'échantillon (114) à l'extérieur du champ de test (116) au moyen d'au moins une mesure optique à résolution temporelle.

7. Dispositif (112) selon la revendication précédente, dans lequel le dispositif de calibration (204) est conçu pour détecter la matière de détection dans l'échantillon (114) à l'extérieur du champ de test (116) au moyen d'une mesure de fluorescence.

8. Dispositif (112) selon l'une quelconque des revendications précédentes, dans lequel l'élément de test (110) comprend au moins une fente capillaire (132) raccordée avec le champ de test (116), pour la réception et la distribution de l'échantillon (114) sur le champ de test (116), le dispositif de calibration (204) étant conçu pour réaliser une calibration d'une largeur de fente de la fente capillaire (132) au moyen de la détection de la matière de détection dans l'échantillon (114) à l'extérieur du champ de test (116).

9. Dispositif (112) selon la revendication précédente, dans lequel la fente capillaire (132) est conçue de telle sorte qu'une couche de l'échantillon (114) d'une épaisseur de couche non supérieure à 50 $\mu$m se forme à l'intérieur de la fente capillaire, le dispositif de calibration (204) étant conçu pour prendre en compte des fluctuations de l'épaisseur de couche de l'échantillon (114)) lors de la détermination de la matière de détection dans l'échantillon (114) à l'extérieur du champ de test (116).

10. Procédé de détection d'au moins un analyte dans un échantillon liquide (114), notamment de détection d'au moins un métabolite dans un liquide corporel, notamment utilisant un dispositif (112) selon l'une quelconque des revendications précédentes concernant un dispositif (112), au moins un élément de test (110) étant utilisé, l'élément de test (110) comprenant au moins un champ de test (116) comprenant une surface de champ de test (124) pour l'application de l'échantillon (114), le champ de test (116) comprenant au moins un réactif de détection (120), qui est conçu pour réaliser une réaction détectable en présence de l'analyte, le réactif de détection (120) étant conçu de telle sorte qu'au moins une matière de détection soit formée lors de la réaction détectable à l'intérieur du champ de test (116), le dispositif étant conçu pour déterminer la matière de détection dans l'échantillon (114) à l'intérieur du champ de test (116), la matière de détection étant également présente à l'extérieur du champ de test (116) dans l'échantillon (114) par diffusion, la matière de détection étant déterminée dans l'échantillon (114) à l'extérieur du champ de test (116), et la détection de l'analyte étant réalisée en prenant en compte la détermination de la matière de détection dans l'échantillon (114) dans le champ de test (116) et la détermination de la matière de détection dans l'échantillon (114) à l'extérieur du champ de test (116), un dispositif de calibration (204) comprenant un appareil de traitement des données étant utilisé, dans lequel des facteurs de correction déposés sont appliqués, qui prennent en compte la diffusion de la matière de détection dans l'échantillon (114) à l'extérieur du champ de test (116).

11. Procédé selon la revendication précédente, dans lequel l'élément de test (110) comprend au moins une fente capillaire (132) raccordée avec le champ de test (116), pour la réception et la distribution de l'échantillon (114) sur le champ de test (116), la fente capillaire (132) étant conçue de telle sorte qu'une couche de l'échantillon (114) d'une épaisseur de couche non supérieure à 50 $\mu$m se forme à l'intérieur de la fente capillaire, des fluctuations de l'épaisseur de couche de l'échantillon (114)) étant prises en compte lors de la détermination de la matière de détection dans l'échantillon (114) à l'extérieur du champ de test (116) .

12. Procédé selon l'une quelconque des revendications de procédé précédentes, dans lequel la détermination de la matière de détection dans l'échantillon (114) à l'extérieur du champ de test (116) a lieu directement, la présence de la matière de détection étant détectée directement qualitativement et/ou quantitativement.

13. Procédé selon l'une quelconque des revendications de procédé précédentes, dans lequel la détermination de ladite au moins une matière de détection dans l'échantillon (114) à l'extérieur du champ de test (116) a lieu indirectement, une diminution de la matière de détection et/ou d'un composé de la matière de détection ou d'une substance contenant la matière de détection étant détectée qualitativement et/ou quantitativement.

Fig. 1

Fig. 2 A

Fig. 2 B

Fig. 3

122, 116, 124

180, 126

Fig. 4 A

180, 126

122, 116, 124

182

Fig. 4 B

Fig. 5 A

Fig. 5 B

Fig. 6 B

Fig. 6 A

124 114

110

Glu    NADH

116, 118, 120

122

GlucDH:NADH    NADH

154, 156, 168

152, 158, 166

Fig. 7

$\Delta t_1$ = 1.5 ns - 2 ns

$\Delta t_2$ = 3.5 ns - 4 ns

$I_{Lumi}$

$t_0$

t (ms)

Fig. 8

Fig. 9 A     Fig. 9 B     Fig. 9 C

EP 2 366 102 B1

Fig. 10

EP 2 366 102 B1

**EP 2 366 102 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0821233 A2
- DE 19753847 A1
- US 20030068666 A1
- EP 0821235 A2
- EP 1733792 A1
- EP 0348006 A2
- US 2002119486 A1
- DE 19844500 A1
- US 4791461 A
- US 2004071331 A1